# EUROPÄISCHE PATENTSCHRIFT

(11) **EP 2 091 518 B1**
(45) Veröffentlichungstag und Bekanntmachung des Hinweises auf die Patenterteilung: **11.09.2013**
(21) Anmeldenummer: 07822344.3
(22) Anmeldetag: 08.11.2007
(51) Int. Cl.: A61K 9/14, A61K 9/16, A61K 47/42

(54) **VERFAHREN ZUR MISCHUNG VON PULVERN**
METHOD FOR MIXING POWDERS
PROCEDE DE MELANGE DE POUDRES

(30) Priorität: 10.11.2006 DE 102006053375
(43) Veröffentlichungstag der Anmeldung: 26.08.2009
(73) Patentinhaber: Boehringer Ingelheim Pharma GmbH & Co. KG, 55216 Ingelheim am Rhein (DE)
(72) Erfinder: SCHULTZ-FADEMRECHT, Torsten, 88437 Maselheim (DE); ZIMONTKOWSKI, Sandra, 72072 Tuebingen (DE); GARIDEL, Patrick, 22846 Norderstedt (DE); KERN, Hans-Joachim, 88441 Mittelbiberach (DE); STEFFENS, Klaus-Jürgen, 53359 Rheinbach (DE)
(74) Vertreter: Simon, Elke Anna Maria
(86) Internationale Anmeldenummer: PCT/EP2007/062040
(87) Internationale Veröffentlichungsnummer: WO 2008/055951

(56) Entgegenhaltungen:
- EP-A- 0 630 651
- EP-A- 1 393 721
- EP-A2- 0 260 971
- WO-A-03/037303
- WO-A-2005/020953
- WO-A-2005/113007
- WO-A-2008/000780

## Beschreibung

### HINTERGRUND DER ERFINDUNG

### TECHNISCHES GEBIET

Die Erfindung betrifft ein Verfahren zur Herstellung von Pulvermischungen, wobei eine Komponente aus sprühgetrocknetem Pulver besteht. Weiterhin betrifft die Erfindung ein Verfahren zum Beschichten von sprühgetrockneten Partikeln mit nanoskaligen Partikeln sowie ein Verfahren zum Belegen von Trägerstoffen mit sprühgetrockneten Partikeln.

### HINTERGRUND

Die Sprühtrocknung ist ein Verfahren, das sehr gut zur Herstellung von inhalierbaren Pulvern geeignet ist. Bei diesem Verfahren können in einem Prozess-Schritt direkt Partikel mit einem MMAD von <10µm hergestellt werden. Alternative Pulververfahren, wie zum Beispiel das Gefriertrocknen oder auch das Präzipitieren benötigen in der Regel einen nachfolgenden Mahl-Schritt.

Ein wesentliches Kriterium für die Güte von inhalierbaren Pulvern sind die Fließfähigkeit sowie die Dispergierbarkeit der Pulver: Besonders kleine Partikel, das heißt, Partikel mit einem MMAD < 10µm, neigen zur Ausbildung stabiler Partikelagglomerate, wodurch die Inhalationseigenschaften der Pulver sich gravierend verschlechtern. Ursache für diese Verschlechterung der Pulvereigenschaften ist die Tatsache, dass bei kleiner werdendem Partikeldurchmesser beispielsweise die Van-der-Waals Kräfte aber auch polare Wechselwirkungen und elektrostatischen Kräfte überproportional ansteigen verglichen zur Gewichtskraft der Partikel.

Das Wissen über dieses Verhalten ist Stand der Technik, so dass vielfältige Lösungsansätze bereits entwickelt wurden.

Beispielsweise können mikronisierte Partikel, d.h. Partikel kleiner 10µm, auf Trägerstoffen aufgezogen werden. Diese Trägerstoffe, zum Beispiel Laktose Monohydrat, Glukose oder Mannitol, haben verglichen zu mikronisierten Partikeln wesentlich größere Partikeldurchmesser (50-200µm), wodurch der Einfluss der Gewichtskraft auf die Partikeleigenschaften zunimmt.

Weitere Strategien zur Verbesserung der Fließ- und Dispergiereigenschaften sind das Erhöhen der Oberflächenrauigkeit zum Beispiel durch ein Beschichten der mikronisierten Partikel mit Nanopartikeln (G.Huber et al., Powder technology 134 (2003) Seiten: 181-192) oder das Hydrophobisieren der Partikel zum Beispiel durch ein Beschichten mit Magnesium Stearat bzw. mit hydrophoben Aminosäuren (WO9200403848).

Bei diesen genannten Verfahren ist nach der Herstellung der mikronisierten Partikel ein zusätzlicher Prozess Schritt notwendig, in dem das entsprechende Pulver mit der zweiten Komponente wie beispielsweise mit einem Trägerstoff, Nanopartikel oder einem Filmbildner vermischt wird.

Während das Vermischen von gemahlenen, kristallinen Mikropartikeln üblich und demnach Stand der Technik ist, ist ein Vermischen von sprühgetrocknetem Pulver eine technische Herausforderung. Sprühgetrocknete Pulver, insbesondere Protein-haltige Pulver, sind in der Regel amorph. Das bedeutet, dass diese Pulver hygroskopisch sind und verglichen zur kristallinen Variante eine höhere Oberflächenenergie und oftmals auch eine höhere elektrostatische Aufladung besitzen. Diese Eigenschaften erschweren gravierend die Mischbarkeit mit einer weiteren Partikelpopulation. Die pharmazeutische Auswirkung eines ungenügenden Mischprozesses ist beispielsweise eine verminderte bzw. ungenügende Homogenität der Dosis des therapeutischen Wirkstoffes bei Anwendung am Patienten.

Ein Verfahren zum Aufziehen von Nanopartikeln auf sprühgetrockneten Pulvern ist das mechanische Beschichten in einer Strahlenmühle beziehungsweise in einem Hybridizer (Firma Nara). Ausserdem möglich ist ein elektrostatisch unterstützes Mischen, wobei die treibende Kraft eine gegengesetzte elektrische Ladung des sprühgetrockneten Partikels und des Nanopartikels ist (G.Huber et al., Powder technology 134 (2003) Seiten: 181-192).

Beim Vermischen von sprühgetrocknetem Material mit Trägersystemen werden überlicherweise Siebe bzw. Freifallmischer eingesetzt.

Bei herkömmlichen Mischverfahren ist es schwierig, eine Prozesskette aufrecht zu halten, die unter reduzierten Luftfeuchten arbeitet. Dieser Aspekt ist im besonderen bei amorphen Pulvern wichtig. Amorphe Pulver neigen speziell bei Lagerung dazu, Wasser aus der Umgebung aufzunehmen. Da aufgenommenes Wasser die Glasübergangstemperatur des Pulvers erniedrigt und somit oftmals die Glasübergangstemperatur unterhalb der Lagertemperatur ist, kommt es zu einer erhöhten Neigung zu Rekristallisations-Effekten. Weiterhin kann es aufgrund einer Wasserdampfsorption am Pulver zu einer Kapillarkondensation des Wasserdampfes kommen, wodurch die Fließfähigkeit des Pulvers sich gravierend verschlechtern kann. Weiterhin kritisch ist bei herkömmlichen zweistufigen Prozessen, dass aktiv in den Prozess eingegriffen werden muss. Das heißt, dass das hergestellte Pulver geerntet wird, um es anschließend der Mischvorrichtung zuzuführen. Dieser Eingriff verschlechtert die Effizienz und Sicherheit des pharmazeutischen Herstellprozesses.

Des weiteren sind die meisten Verfahren, wie z.B. das Mischen in einer Strahlenmühle oder Kugelmühle aber auch im besonderen im Hybridizer, durch die mechanische Belastung mit Temperatureffekten ("hot spots") verbunden. Ähnlich wie bei der Wasseraufnahme kann es durch das Überschreiten der Glasübergangstemperatur des Pulvers lokal zu Rekristallisation des amorphen, sprühgetrockneten Pulvers kommen. Durch die mechanischen Belastungen beim Mischen können ausserdem die sprühgetrockneten Partikel durch Bruch oder Aufschmelzung zerstört werden.

Eine weitere Problemstellung ergibt sich aus der Zerstörung der Feinstruktur von nanoskaligen Partikeln durch die mechanische Belastung sowie durch die Einwirkdauer der mechanischen Belastung bei Mischprozessen. Die Nanopartikel fungieren auf einem sprühgetrocknetem Partikel als Abstandshalter und verbessern durch die Reduktion der Van-der-Waals Kräfte die Fließfähigkeit der Pulver sowie das aerodynamische Verhalten. Bei hoher Belastungsintensität kann sich auf einem Partikel ein geschlossener Film bestehend aus den Nanopartikeln ausbilden und so den positiven Effekten entgegenwirken. (M.Eber, 2004, Disseratation Uni Erlangen, Titel: Wirksamkeit und Leistungsfähigkeit von Nanoskaligen Flussregulierungsmitteln)

Eine Aufgabenstellung der vorliegenden Erfindung ist daher die Verbesserung eines Mischverfahrens bei reduzierten Luftfeuchten.

Eine weitere Aufgabenstellung ist die Verringerung der mechanischen Belastung und damit von "hot spots" beim Mischverfahren.

Die veröffentlichte Patentanmeldung WO03/037303 beschreibt ein Verfahren bei welchem aus sprühgetrockneten Pulvern in einem einstufigen Prozess Partikelmischungen hergestellt werden und zwar durch das gleichzeitige Sprühen zweier Sprühmedien in einen Trocknungsturm. Hiernach wird über eine zweite Zerstäubungsdüse eine zusätzliche Partikelpopulation erzeugt, die sich anschließend im Sprühtrockner mit dem wirkstoffhaltigen Pulver vermischt.

Der Nachteil bei diesem Verfahren ist, dass mit dieser Technologie lediglich ein sehr reduziertes Größensprektrum der beiden Partikelpopulationen möglich ist. So wird es nicht möglich sein, sprühgetrocknete, wirkstoffhaltige Partikel mit nanoskaligen Partikeln zu beschichten. Außerdem können mit diesem Verfahren keine Mischungen aus einem kristallinen und damit auch thermodynamisch stabilen Trägerstoff (mit einer bevorzugten Größe zwischen 50-200µm) und sprühgetrockneten Pulvern hergestellt werden.

Eine weitere Aufgabenstellung der vorliegenden Erfindung ist daher das Vermischen der sprühgetrocketen Partikel mit Nanopartikeln bzw. mit kristallinen Trägerstoffen.

Bei der Sprühtrocknung thermolabiler Wirkstoffe, insbesondere von Proteine, sowie bei der Herstellung von Pulvern mit einer niedrigen Glasübergangstemperatur ergeben sich zusätzliche Probleme, da durch eine ungünstige Temperaturführung bei der Sprühtrocknung das Protein geschädigt werden kann.

Unter diesen Voraussetzungen muss bei der klassischen Sprühtrocknung die Auslasstemperatur reduziert werden, wodurch der gesamte Trocknungsprozess suboptimal gefahren werden muss.

Ein mögliche Strategie zur Optimierung des Trocknungsprozesses ist das Kühlen des Zyklons. Nachteilig bei diesem Verfahren ist jedoch, dass Pulverablagerungen im Zyklon isolierend wirken und daher die Kühlung träge und ineffizient werden kann.

Eine weitere Aufgabenstellung der vorliegenden Erfindung betrifft daher die notwendige und schnelle bzw. effiziente Begrenzung der Auslasstemperatur bei der Sprühtrocknung unter Beibehalt hoher Einlasstemperaturen.

Das Herstellen von Pulver mit einer hohen Proteinbeladung, z.B. größer 50% (w/w), ist im besonderen problematisch. Hohe Proteinanteile verschlechtern die Pulvereigenschaften, wie zum Beispiel die Fließfähigkeit, gravierend. Die Pulver zeigen in der Regel eine sehr hohe Neigung zur Kohäsion sowie zur Adhäsion. Das bedeutet, dass die Ausbeuten des Trocknungsprozesses sowie der weiter folgenden Prozessschritte beeinträchtigt werden. Außerdem zeigen Pulver mit hohen Anteilen an therapeutischen Proteinen aufgrund des kohäsiven Charakters der Proteine eine schlechte Inhalierbarkeit. Hieraus ergibt sich die Problemstellung, sprühgetrocknete Pulver mit hohen Anteilen an therapeutischen Proteinen ohne Verschlechterungen in den Pulver- und Inhaliereigenschaften herzustellen.

Ein komplett anderer Ansatz zur Optimierung von physikochemischen Eigenschaften sprühgetrocketer Pulver ist die Veränderung der Pulverzusammensetzung. So vermag der Fachmann durch Zugabe von hydrophoben Substanzen in die Sprühlösung, die Partikeloberfläche der resultierenden Partikel zu modifizieren, um so bessere Dispergiereigenschaften zu erhalten. Nachteilig ist jedoch, dass es zu Inkompatibilität der hydrophoben Hilfsstoffe mit dem Wirkstoff kommen kann. So ist es beispielsweise bekannt, dass hydrophobe Hilfsstoffe eine höhere Affinität zu denaturierten als zu nativen Proteinen hat. Durch eine Interaktion von hydrophoben Hilfsstoffen und Proteinen können daher Proteinaggregate induziert werden.

Weiterhin nachteilig ist, dass hydrophobe Substanzen im sprühgetrockneten Partikel oftmals unerwünschte Kristallisationseffekte hervorrufen, wodurch ebenfalls eine Proteinschädigung hervorgerufen werden kann.

Hieraus ergibt sich das zusätzliche Problem, dass eine hinreichende Fließfähigkeit bzw. ein hinreichendes Dispergierverhalten sprühgetrockneter Pulver ohne Schädigung des Proteins bedingt durch Kristallisationseffekte der Pulver sicher gestellt werden muss.

EP0630651 beschreibt halbfeste pharmazeutische Agenzien wie Zäpfchen, Salben, Pasten sowie die Mischung flüssiger Lösungen und deren anschließende Trocknung. Das resultierende Pulver, welches das bioaktive Protein enthält, wird mit einer wasserlöslichen Basis oder einer Emulsion oder einen Öl oder Fett vermischt.

WO2008/000780 beschreibt die Herstellung von Pulvern mit einem Proteinanteil und einem Phenylalaninanteil. Hierbei wird die Mischung von wässrigen Lösungen beschrieben, die zusammen versprüht werden. Das resultierende Pulver ist das Endprodukt aber nicht die Ausgangssubstanz.

EP0260971 beschreibt ein Sprühtrocknungsverfahren bei der Waschmittelherstellung, bei welchem zwei Flüssigkomponenten simultan im Sprühtrockner versprüht werden. Zur Verbesserung der Herstellung von Detergenz-Granulaten wird hierbei zeitgleich mit der Detergenz-Suspension auch Alkalimetalsilikat eingesprüht.

Es ergibt sich somit die Aufgabe ein Verfahren zur Herstellung von Pulvermischungungen bestehend aus sprühgetrockneten Pulvern und Trägerstoffen beziehungsweise mit nanoskaligen Partikeln bereit zu stellen, welches
1. kontinuierlich eine reduzierte Luftfeuchte ermöglicht,
2. eine Verringerung der mechanischen Belastung darstellt,
3. besonders schonend für die Partikel sowie für die Wirkstoffstabilität ist und zudem
4. eine hinreichende Fliessfähigkeit bzw. ein hinreichendes Dispergierverhalten der sprühgetrockneten Pulver sicher stellt.

### ZUSAMMENFASSUNG DER ERFINDUNG

Die vorliegende Erfindung löst die Aufgabe durch ein Verfahren zur Mischung von sprühgetrockneten Pulver mit Nanopartikel, mikroskaligen Partikeln oder Trägerstoffen, welches direkt im Sprühtrockner in einem einstufigen Prozess durchgeführt wird. Dies ist für das sprühgetrocknte Pulver ein sehr schonendes Verfahren. Da das Vermischen direkt im Sprühtrockner erfolgt, kann der Mischprozess bei sehr niedrigen Luftfeuchten gefahren werden. Ein Transfer des Pulvers nach Sprühtrocknung in eine Mischapparatur entfällt. Ein zusätzlicher Energieeintrag zum Dispergieren des sprühgetrockenten Pulvers ist nicht notwendig, da beim Sprühtrocknen durch die vorherige Zerstäubung der Sprühlösung das entstehende Pulver optimal dispergiert vorliegt.

Durch das schonende Dispergieren der Nanopartikel kommt es des weiteren kaum zu Zerstörungen der gewünschten Nanostrukturen.

Zusätzlich kann man durch das Einblasen von kühler Luft nach Abschluss der Trocknung die Temperaturbelastung des sprühgetrockneten Pulvers reduzieren. Hierdurch wird das Pulver weniger belastet, was besonders bei längeren Prozesszeiten von Vorteil ist.

Bei der Entwicklung von sprühgetrockneten Pulvern, und im besonderen bei Proteinhaltigen Pulverrezepturen, hat der Fachmann in der Regel das Problem, sowohl eine gute Wirkstoffstabilität als auch gute Pulvereigenschaften (wie z.B. die Fließfähigkeit und Dispergierbarkeit) zu erzielen. Speziell bei hohen Proteinanteilen im Pulver, wie sie z.B. bei hoch zu dosierenden Arzneistoffen notwendig ist, neigen die Pulver zu einem sehr stark kohäsiven Verhalten. Typischer Vertreter dieser Wirkstoffklasse sind Antikörper des Typs IgG.

Durch die vorliegende Erfindung ist es nun möglich, die Abhängigkeit zwischen Proteinstabilisierung und Pulvereigenschaften weitgehend zu entkoppeln. Die Rezepturfindung der Sprühlösung und damit des Pulvers kann im Wesentlichen auf die Proteinstabilität fokussiert werden. Die Optimierung der aerodynamischen Eigenschaften ist dagegen durch ein Vermischen mit geeigneten Hilfsstoffen direkt im Sprühtrockner realisierbar.

Des weiteren kann durch ein Zumischen eines inerten Hilfsstoffes der Proteinanteil in der Pulvermischung und damit die Wirkstoffdosierung reguliert werden. Das Herstellen verschiedener Dosierungen wird durch ein Vermischen mit einem Trägerstoff wesentlich vereinfacht.

Hieraus ergeben sich außerdem reduzierte Prozesszeiten und weniger Kosten für die Bereitstellung geeigneter Maschinen.

Durch die Option des Kühlens des Pulvers bedingt durch das Einblasen von kühler Luft nach Trocknung der Sprühlösung kann das Pulver weiter stabilisiert werden, so dass auch thermolabilere Substanzen besser prozessierbar sind.

Eine Anwendung der Erfindung ist die Entwicklung von Pulvern, z.B. pulverhaltiger Darreichungsformen von Arzneimitteln z.B. für die Inhalation sowie für nasale oder orale Applikationen. Eine weitere Anwendung der entwickelten Pulver ist das Auflösen der Pulver (Rekonstitution) in einer geeigneten Flüssigkeit mit anschließender intravenöser, subkutaner, intramuskulärer oder intraartikulärer Applikation.

Eine besonders vorteilhafte Ausführungsform des erfindungsgemäßen Verfahrens ist die Dosiseinstellung der Pulvermischung mit mikroskaligen Mischungskomponenten. Hierbei sollte die Partikelgröße kleiner 10µm beziehungsweise kleiner 5µm sein. Idealerweise sollte die Partikelgröße der Mischungskomponente in der gleichen Größenordnung sein wie das sprühgetrocknete Pulver.

Eine weitere besonders vorteilhafte Ausführungsform des erfindungsgemäßen Verfahrens ist die Dosiseinstellung der Pulvermischung mit Trägerstoffen. Bei Herstellung von Pulver für die inhalative Anwendung sollte der Trägerstoff einen hohen Anteil (größer 30%) an Partikel mit einer Partikelgröße kleiner 100µm haben (siehe Beispiel 7 Tabelle 13).

Die Zumischung von nanoskaligen Partikeln in den Sprühtrockner stellt eine besondere Herausforderung dar, weil konventionelle Dosiervorrichtungen wie beispielsweise Dosierschnecke, Banddosierer, Bürstendosierer (Firma Palas), Vibrationsrinnen etc. für nanoskalige Partikel ungeeignet sind. Daher besteht eine weitere besonders vorteilhafte Ausführungsform des erfindungsgemäßen Verfahrens in der Zumischung von nanoskaligen Partikeln in den Sprühtrockner. Die Einstellung des Massenstromes in den Sprühtrockner erfolgt erfindungsgemäß pneumatisch. In dieser Ausführungsform wird in einem Vorratsgefäß das Pulverbett der Mischungskomponente durch mechanisches Rühren homogenisiert. Durch einen Luftstrom werden die nanoskaligen Partikel ins Aerosol überführt und anschließend über eine Venturidüse in den Sprühtrockner eingespeist. Der Massenstrom wird sowohl durch den Energieeintrag beim mechanischen Rühren als auch durch den Volumenstrom im Vorratsgefäß eingestellt.

Eine weitere besonders vorteilhafte Ausführungsform des erfindungsgemäßen Verfahrens ist das Vermischen des sprühgetrockneten Pulvers mit hydrophilen beziehungsweise mit hydrophoben nanoskaligen Partikeln. Beispiele für geeignete nanoskalige Partikel sind hochdisperses hydrophiles Siliziumdioxid oder das hydrophobisierte Aerosil R972. Der Einsatz der nanoskaligen Partikeln sind nicht auf die genannten Siliziumdioxide beschränkt. Maßgeblich für die Einsetzbarkeit der Hilfsstoffe ist die Partikelgröße, die wesentlich kleiner als 1000nm, beziehungsweise kleiner 500nm und besonders vorteilhaft kleiner 100nm sein sollte.

Die Erfindung ergibt sich nicht aus dem Stand der Technik.

In der veröffentlichten Patentanmeldung WO200403848 wurden Pulver (auch sprühgetrocknete Pulver) nach Herstellung mit einer Aminosäure, mit Mg-Stearat sowie mit einem Phospholipid in einer Mühle (Strahlenmühle/Kugelmühle) vermischt. Ziel dieser Vorgehensweise war die Optimierung der aerodynamischen Eigenschaften von Pulver durch das Modifizieren der Partikeloberflächen (Hydrophobisierung). Ziel dieses Verfahrens ist die Ausbildung von Filmen auf der Partikeloberfläche und nicht die Erhöhung der Oberflächenrauigkeit. Die Erhöhung der Oberflächenrauigkeit ist dagegen ein wichtiger Aspekt der vorliegenden Erfindung. Des weiteren ist mit dem in der Patentanmeldung beschriebenen Verfahren eine Kombination der Herstellung der sprühgetrockneten Pulver mit der Oberflächenmodifizierung in einem einstufigen Prozess nicht möglich.

In den veröffentlichten Patentanmeldungen WO2000053158 und WO2000033811 werden Mischverfahren beschrieben bei denen ein bereits hergestelltes Pulver durch einen weiteren Mischvorgang mit einem zusätzlichen Pulver vermischt wird. Ziel dieser Mischvorgänge ist das Optimieren von Pulvereigenschaften sowie das Aufziehen des Pulver auf einen Trägerstoff. Als Mischer werden Siebe und Freifallmischer verwendet (z.B Turbular Mischer). Weiterhin genannt werden Mischprinzipien, wonach die Mischung durch Scherstress induziert wird. Die in den Patentanmeldungen genannten Verfahren sind jedoch stets zweistufige Prozesse. Das heißt im Gegensatz zur vorliegenden Erfindung erfolgt die Mischung nach Pulverherstellung in einem separaten Prozessschritt.

In einer weiteren veröffentlichten Patentanmeldung (US2004/0118007, "Methods and Apparatus for making particles using spray dryer and in-line jet milling") wird ein Verfahren beschrieben, bei dem das sprühgetrocknete Pulver direkt nach Herstellung in-line in eine Strahlenmühle eingespeist wird. Ziel dieses zusätzlichen Schrittes ist das Zerstören von Agglomeraten, aber nicht die Oberflächenmodifikation der sprühgetrockneten Partikel durch die Zugabe von Trägersystem und Nanopartikeln. Mit diesem Verfahren ist es ausserdem nicht möglich, Dosierungen durch Verdünnen mit Trägerstoffen herzustellen.

Die veröffentlichte Patentanmeldung WO03/037303 beschreibt ebenfalls ein Verfahren, bei dem direkt im Sprühtrockner eine Pulvermischung hergestellt wird. Bei diesem Verfahren werden über eine Mehrfachdüse 2 Sprühlösungen unabhängig voneinander in den Trocknungsturm eingespeist. In einer Herstellung werden sowohl Raffinose- als auch Leucin- Partikel hergestellt. Die Partikel mischen sich anschließend im Sprühtrockner. Mit diesem Verfahren können jedoch keine Mischungen mit kristallinen Trägersystemen und Nanopartikeln hergestellt werden.

Das Dokument GB866038 beschreibt ein Verfahren zur Herstellung von Polyvinylazetat-Pulver durch Sprühtrocknung. Bei diesem Verfahren wird ein inertes Pulver, zum Beispiel Calcium Carbonat oder Titandioxid, auch als Mischung mit einem so genannten Plasticizer während des Trocknungsprozesses eingebracht. Wesentlich an diesem Verfahren ist, dass der Trocknungsprozess des Polyvenylazetats erst nach Einspeisung des inerten Materials abgeschlossen ist. Ziel ist es, das inerte Material im sprühgetrockneten Partikel einzukapseln, aber nicht durch die Einspeisung des inerten Partikels die Oberflächenbeschaffenheit zu modifizieren. Des weiteren handelt es sich bei diesem Verfahren nicht um ein Mischverfahren zweier Partikelpopulation, sondern um die Herstellung von neuen hybridartigen Partikeln bestehend aus Polyvinylazetat und dem interten Material.

Das Patent US3842888 beschreibt ein Verfahren zur Herstellung von Borax Pentahydrat. Bei diesem Verfahren wird im Gegenstromverfahren in der Weise getrocknet, dass zuerst eine Detergenzienlösung im Trocknungsturm vorgetrocknet wird und dass die zu trocknende Borax-Lösung anschließend unterhalb der Zuführung der Detergenzienlösung in den Trocknungsturm eingespeist wird. Ziel dieses Verfahrens ist die Herstellung von Pulver, im wesentlichen Borax, mit einer geringen Dichte. Bei diesem Verfahren werden keine Mischungen aus zwei verschiedenen Pulver hergestellt. Des weiteren beinhaltet dieses Patent keine Einspeisung eines Pulvers in den Trocknungsturm, sondern die Einspeisung zweier Flüssigkeiten.

Das Dokument JP8302399 beschreibt ein Verfahren, wonach im Gegenstromverfahren Detergenzien getrocknet werden. Zur Verbesserung der Ausbeute der getrockneten Detergenzien-Granulate wird ein anorganischer Hilfsstoff, zum Beispiel ein Aluminiumsilikat, in den Trocknungsturm eingespeist. Dieses Verfahren unterscheidet sich von der vorliegenden Erfindung dahingehend, dass mit diesem Verfahren Detergenzien im Gegenstromverfahren und nicht im Gleichstromverfahren getrocknet werden. Speziell thermolabile Wirkstoffe werden bei einer Gegenstromtrocknung bedingt durch die stärkere Temperaturexposition geschädigt. Bei Proteinen kann es beispielsweise zu Denaturierungen kommen. Zudem beschreibt das Dokument lediglich die Sprühtrocknung von Detergenzien. Die Trocknung von pharmazeutischen Wirkstoffen ist nicht Bestandteil des Dokumentes. Die Bedingungen für Detergenzien sind auf Proteine oder pharmazeutische Wirkstoffe insbesondere Antikörper nicht ohne weiteres übertragbar, da diese thermolabiler und empfindlicher sind.

### BESCHREIBUNG DER ABBILDUNGEN

Sämtlich in den Beschreibungen aufgeführten Prozentangaben beziehen sich auf Konzentrationsangaben und Zusammensetzungen der trockenen Feststoffe insbesondere in einem durch Sprühtrocknung erzielten Pulver (w/w).

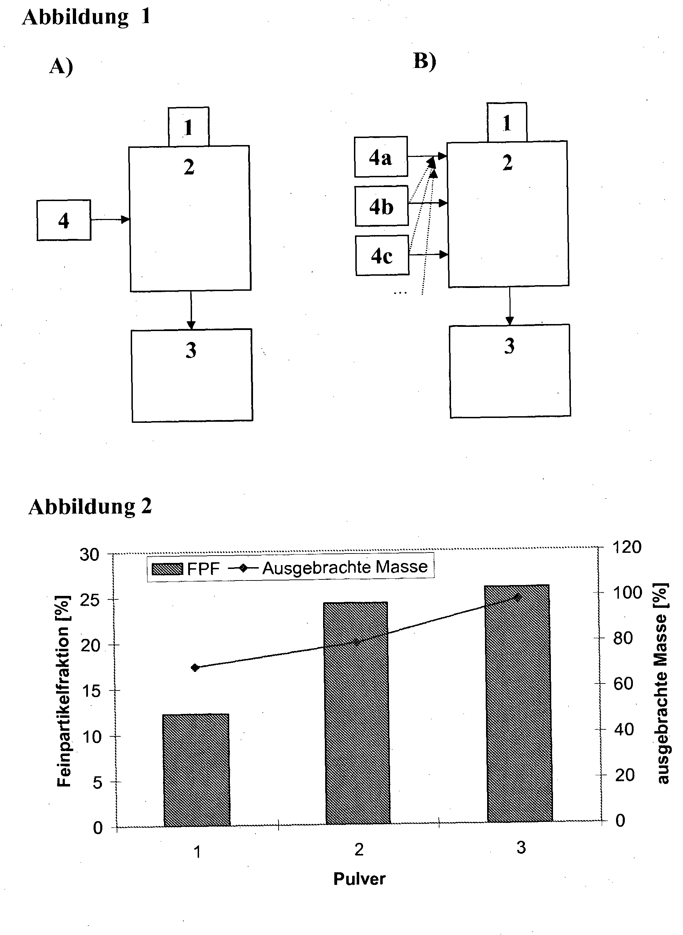

### ABBILDUNG 1: SKIZZE MODIFIZIERTER SPRÜHTROCKNUNGSPROZESS MIT INTEGRIERTER MISCHEINHEIT

Abbildung 1 zeigt das Schema für einen modifizierten Sprühtrocknungsprozess. Eine Sprühlösung, enthaltend einen oder mehrere Wirkstoffe und einen oder mehrere Hilfsstoffe werden über eine Pumpvorrichtung der
- Zerstäubungseinheit (1) zugeführt. Hierbei kann es sich um ein beliebiges Zerstäubungssystem handeln, z.B. Zweistoff- bzw. Dreistoffdüsen, Druckdüsen, Zentrifugaldüsen, Venturidüsen oder Ultraschalldüsen. Nach der Zerstäubung wird der entstandene Tropfen durch ein Trocknungsgas im
- Trocknungsturm (2) im Gleichstromverfahren eingedampft bis schließlich ein Partikel entstanden ist. Nach dem Trocknungsprozess wird über eine (siehe Abbildung 1A) oder mehrere (siehe Abbildung 1 B) geeignete
- Dispergier- und Zudosierungseinheiten (4) ein (siehe Abbildung 1A) oder mehrere (siehe Abbildung 1B) weitere Pulver in den Trocknungsturm (2) eingebracht. Im Trocknungsturm (2) sowie in dem anschließenden
- Partikelsammler bzw. Gas/Feststoff Separator (3), z.B. ein Zyklon, verbinden sich die beiden verschiedenen Pulver und bilden eine Mischung. Die Mischung kann zum Beispiel aus dem sprühgetrocknetem Pulver und einem Pulver aus nanoskaligen Partikeln oder aus sprühgetrocknetem Pulver und einem Trägerstoff, z.B. kristallinem Laktose Monohydrat bestehen. Auch Kombinationen aus sprühgetrockneten Pulver, nanoskaligen Partikeln und /sowie Trägerstoffen sind Bestandteil der Erfindung. Eine weitere bevorzugte Ausführungsform ist das Vermischen verschiedener sprühgetrockneter Pulver.
   A) Zuführung von einem weiteren Pulver / Trägerstoff /Nanopartikel
   B) Zuführung von mehreren weiteren Pulvern, Trägerstoffen bzw. Nanopartikeln angedeutet als 4a, 4b und 4c. Gestrichelte Pfeile deuten eine Verfahrensvariante an, bei der diese mehreren weiteren Pulver, Trägerstoffe bzw. Nanopartikel zunächst vorgemischt werden und dann über eine einzige geeignete Dispergier- und Zudosierungseinheit zugeführt werden.

Die Trocknung des Tropfens und die Generierung des sprühgetrockneten Pulvers entspricht dem aktuellen Stand der Technik. Das heißt, die Sprühlösung kann sowohl wässrig sein als auch aus einem belieben, pharamzeutisch akzeptablen organischen Lösungmittel bestehen. Das Trocknungsmedium ist entweder Luft oder Stickstoff. Die Eingangstemperaturen des Trocknungsgases in den Trocknungsturm liegen zwischen 50°C und 200°C. Die Auslasstemperaturen nach dem Passieren des Trocknungsturms liegen zwischen 25°C und 150°C.

### ABBILDUNG 2: FEINPARTIKELFRAKTION (FPF) UND AUSGEBRACHTE MASSE VERSCHIEDENER PULVER (MIT/OHNE GRANULAC 140)

Die Feinpartikelfraktion wurde mit einem Einstufenimpaktor (Impactor Inlet, TSI) in Kombination mit dem Aerodynamic Particle Sizer (APS, TSI) bestimmt. Die Trenngrenze der Impaktordüse lag bei 5,0 µm.

Für die Messung wurde das Pulver in Kapseln der Größe 3 abgefüllt und mit einem Inhalator (HandiHaler®, Boehringer Ingelheim) ausgebracht. Die Flussrate zur Ausbringung des Pulvers wurde so eingestellt, dass über den HandiHaler ein Druckabfall von 4 kPa vorherrschte. Das Luftvolumen betrugt entsprechend der PharmEur 4 Liter. Um ein "Rebouncing" der an der Impaktorstufe abgeschiedenen Partikel zu vermeiden, wurde bei den Messungen die Impaktorplatte mit einer hochviskosen Brij-Lösung beschichtet.

Die ausgebrachte Masse ergibt sich aus der Differenzwiegung der Kapsel vor und nach Ausbringung des Pulvers aus dem Inhalator.

Die Feinpartikelfraktion wurde nasschemisch ermittelt. Hierzu wurde der Filter, auf der die Feinpartikelfraktion abgeschieden wurde, für 3 Minuten in einem Rekonstitutionsmedium unter leichtem Schwenken inkubiert. Anschließend wurde das Rekonstitutionsmedium sterilfitriert und die Proteinkonzentration im Filtrat mit UV-Spektroskopie bestimmt. Die dargestellten Ergebnisse ergeben sich aus drei Einzelbestimmungen.

Die Balken 1, 2 und 3 stehen für die drei hergestellten Pulvermischungen. Der Mischungsanteil von Granulac 140 lag beim Pulver 1 bei 0% (w/w), beim Pulver 2 bei 30% (w/w) und beim Pulver 3 bei 90%.

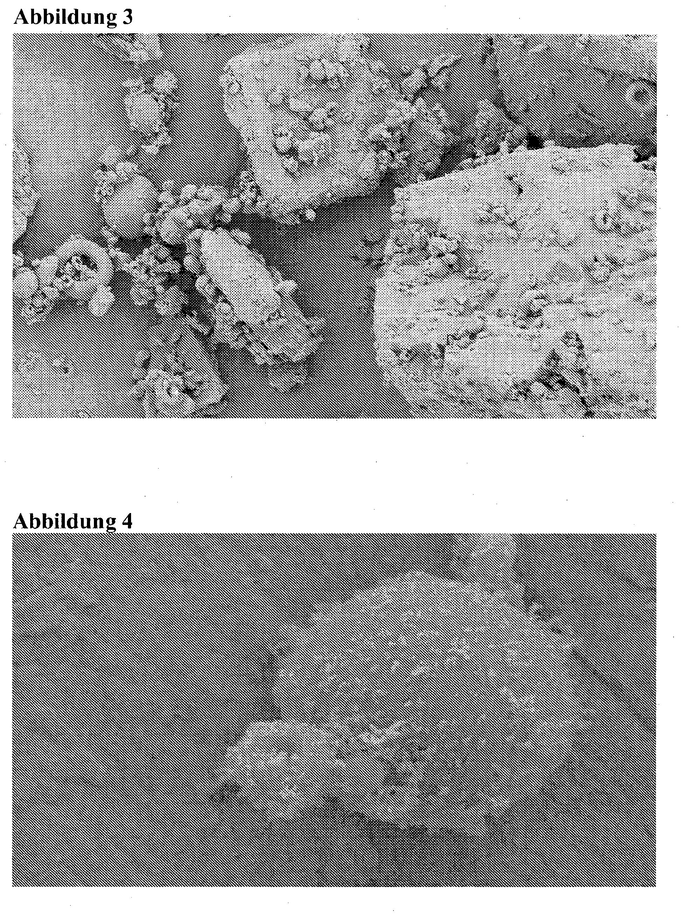

ABBILDUNG 3: REM BILD - MISCHUNG AUS SPRÜHGETROCKNETEM PULVER UND EINEM TRÄGERSTOFF REM Aufnahme von sprühgetrocknetem Pulver enthaltend einen 70 % (w/v) IgG1 Antikörper und 30% (w/v) Trehalose und dem Trägermaterial Granulac 140. (kristallines Laktose Monohydrat).

Zusammensetzung der Mischung: 30% sprühgetrocknetes Pulvers / 70% Granulac 140

Die Aufnahmen wurden mit einem Rasterelektronenmikroskop (SUPRA 55 VP, Fa. Zeiss SMT, Oberkochen) aufgenommen. Hierzu wurden die Pulverproben direkt auf geeignete Probenteller aufgestäubt. Überschüssiges Material wurde abgeklopft und abgeblasen. Anschließend wurden die Proben zur Sicherstellung einer ausreichenden elektrischen Leitfähigkeit mit 15 nm Gold-Palladium beschichtet.

Die Detektion zur Darstellung der Bilder erfolgte über Sekundärelektronen.
Vergrößerung: 1000 fach
Abstand Pulver-Kathode: 10mm
Blendengröße: 10 µm
Beschleunigungsspannung: 5 kV
Vakuum: 4,17e-004 Pa

ABBILDUNG 4 REM BILD - MISCHUNG AUS SPRÜHGETROCKNETEM PULVER UND NANOPARTIKEL REM Aufnahme von sprühgetrocknetem Pulver enthaltend einen 70 % (w/v) IgG2 Antikörper und 30% (w/v) Trehalose und dem Aerosil R812. Aerosil R812 besteht aus nanoskaligen, hydrophobisiertem Siliciumdioxid

Zusammensetzung der Mischung: 66% sprühgetrocknetes Pulvers / 24% Aerosil 812R

Die Aufnahmen wurden mit einem Rasterelektronenmikroskop (SUPRA 55 VP, Fa. Zeiss SMT, Oberkochen) aufgenommen. Hierzu wurden die Pulverproben direkt auf geeignete Probenteller aufgestäubt. Überschüssiges Material wurde abgeklopft und abgeblasen. Anschließend wurden die Proben zur Sicherstellung einer ausreichenden elektrischen Leitfähigkeit mit ca. 15 nm Gold-Palladium beschichtet.

Die Detektion zur Darstellung der Bilder erfolgte über Sekundärelektronen.
Vergrößerung: 15000 fach
Abstand Pulver-Kathode: 5mm
Blendengröße: 10 µm
Beschleunigungsspannung: 5 kV
Vakuum: 4,24e-004 Pa

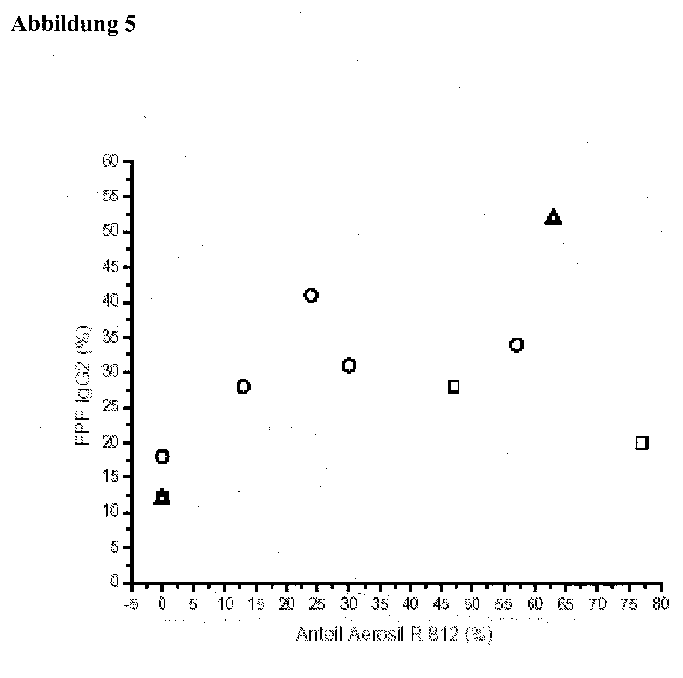

ABBILDUNG 5: FEINPARTIKELFRAKTION (FPF) IN ABHÄNGIGKEIT DES MISCHUNGSANTEILS AN NANOPARTIKELN UND DES DISPERGIERDRUCKES Dieses Diagramm zeigt verschiedene Pulvermischungen bestehend aus sprühgetrocknetem Pulver (70% (w/v) IgG2 / 30%(w/v) Trehalose) und Aerosil R812. Die Pulver wurden bei verschiedenen Drücken dispergiert und in den Sprühtrockner eingebracht.

Die Sprühtrocknungen erfolgten an einem Büchi B191. Es wurden folgende Sprühtrocknungsbedingungen eingestellt:

| | |
|---|---|
| Eingangstemeraptur: | 150°C |
| Ausgangstemerpatur: | 90°C |
| Zerstäubergasrate: | 700L/h |
| Aspiratorleitstung: | 100% |
| Sprührate: | 3ml/min |
| Feststoffgehalt der Sprühlösung: | 3% |
| Quadrat: | Dispergierdruck 0,5 bar |
| Kreis: | Dispergierdruck 1,75 bar |
| Dreieck: | Dispergierdruck 3,0 bar |

### DETAILLIERTE BESCHREIBUNG DER ERFINDUNG

Im Rahmen dieser Erfindungsbeschreibung verwendete Begriffe und Bezeichnungen haben folgende im Anschluss definierte Bedeutungen. Die Gewichts- und Gewichtsprozentangaben beziehen sich, sofern nichts anderes erwähnt wird, jeweils auf die Trockenmasse der Zusammensetzungen oder den Feststoffgehalt der Lösungen / Suspensionen. Die allgemeinen Ausführungsformen "enthaltend" oder "enthält" schließt die speziellere Ausführungsform "bestehend aus" mit ein. Ferner werden "Einzahl" und "Mehrzahl" nicht begrenzend verwendet.

"Pulver" bezeichnet einen sehr feinen, zerkleinerten Stoff.

"Sprühgetrocknete Pulver" meint ein Pulver, welches mittels Sprühtrocknung hergestellt wurde.

"Partikel" bezeichnet ein Teilchen von einem Stoff. In der vorliegenden Erfindung sind mit Partikeln die Partikel in den erfindungsgemäßen Pulvern gemeint. Die Begriffe Partikel und Pulver werden in der vorliegenden Erfindung zeitweise austauschbar verwendet. Mit einem Pulver sind auch dessen Bestandteile, die Partikel, umfasst. Partikel weisen auch auf die Gesamtmenge von Partikeln also das Pulver hin.

Der Ausdruck "Zusammensetzung", meint flüssige, halbfeste oder feste Gemische aus zumindest zwei Ausgangsstoffen.

Der Begriff "pharmazeutische Zusammensetzung" meint eine Zusammensetzung zur Applikation im Patienten.

Der Begriff "pharmazeutisch akzeptable Hilfsstoffe" bezieht sich auf Hilfsstoffe, die optional im Rahmen der Erfindung in der Formulierung enthalten sein können. Die Hilfsstoffe können beispielsweise pulmonal appliziert werden ohne hierbei signifikant ungünstige toxikologische Effekte auf den Probanden oder die Probandenlunge zu haben.

Der Begriff "Mischen" meint einen Prozess bei dem verschiedene Pulver möglichst homogen verteilt vereint werden. Bei diesem Prozess können die zu mischenden Partikel gleiche aber auch verschiedene mittlere Partikelgrößen aufweisen. Beispielsweise beinhaltet der Begriff Mischen das Vereinen von zwei sprühgetrockneten Pulver. Weiterhin beinhaltet der Begriff Mischen das Vereinen von sprühgetrockneten Pulvern mit nanoskaligen Partikeln oder mit Trägerstoffen. Der Begriff Mischen umfasst demnach auch das Beschichten eine Partikelpopulation mit einer zweiten Partikelpopulation.

Der Begriff "Plasticizer" beschreibt eine Stoffeigenschaft, wonach dieser Stoff die Glasübergangstemperatur eines amorphen Pulvers erniedrigt. So ist beispielsweise Wasser ein Plasticizer für sprühgetrocknete Pulver und erniedrigt entsprechend dem Feuchtegehalt des Pulvers die Glasübergangstemperatur.

Ein einstufiger Prozess grenzt sich von einem zweistufigen Prozess dadurch ab, dass in einer Einheit, z.B. in einem Gerät, in einer Kammer oder Ähnlichem zwei Prozess- bzw. Verfahrensschritte erfolgen. Bei einem zweistufigen Prozess werden für 2 Verfahrensschritte auch 2 Einheiten benötigt.

Besteht beispielsweise ein Verfahren aus einem Trocknungsschritt und einem Mischschritt, so würde bei einem einstufigen Verfahren sowohl die Trocknung als auch das Mischen in einem Gerät beziehungsweise einer Einheit erfolgen. Diese Einheit kann zum Beispiel ein Sprühtrockner sein. Bei einem zweistufigen Prozess wurde das Pulver nach der Trocknung vom ersten Gerät für den folgenden Mischprozess in ein zweites Gerät überführt.

Der Ausdruck "pharmazeutisch akzeptable Salze" umfasst beispielsweise folgende Salze, ist aber nicht begrenzt auf diese: Salze aus anorganischen Säuren, wie Chlorid, Sulfat, Phosphat, Diphosphat, Bromid und Nitratsalze. Des weiteren Salze aus organischen Säuren, wie Malat, Maleat, Fumarat, Tartrat, Succinat, Ethylsuccinat, Citrat, Acetat, Lactat, Methansulfonat, Benzoat, Ascorbat, Para-Toluolsulfonat, Palmoat, Salicylat und Stearat, ebenso wie Estolat, Gluceptat und Lactobionat Salze.

Mit dem Begriff "Wirkstoffe" sind Substanzen gemeint, die in einem Organismus eine Wirkung bzw. eine Reaktion hervorrufen. Wird ein Wirkstoff zu therapeutischen Zwecken am Menschen oder am tierischen Körper angewandt so bezeichnet man ihn als Arzneimittel oder Medikament.

Beispiele für Wirkstoffe sind Insulin, Insulin-ähnlicher Wachstumsfaktor, humanes Wachstumshormon (hGH) und andere Wachstumsfaktoren, Gewebeplasminogenaktivator (tPA), Erythropoetin (EPO), Cytokine, beispielsweise Interleukine (IL) wie IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, Interferon (IFN)-alpha, beta, gamma, omega oder tau, Tumornekrosefaktor (TNF) wie z.B. TNF-alpha, beta oder gamma, TRAIL, G-CSF, GM-CSF, M-CSF, MCP-1 und VEGF. Weitere Beispiele sind monoklonale, polyklonale, multispezifische und einzelkettige (*single chain*) Antikörper und Fragmente davon, wie z.B. Fab, Fab', F(ab')₂, Fc und Fc'-Fragmente, leichte (L) und schwere (H) Immunglobulinketten und deren konstante, variable oder hypervariable Regionen sowie Fv- und Fd-Fragmente (Chamov et al., 1999). Die Antikörper können humanen oder nicht-humanen Ursprungs sein. Auch humanisierte und chimäre Antikörper kommen in Frage. Ebenso betrifft dies konjugierte Proteine und Antikörper, welche beispielsweise mit einer radioaktiven Substanz oder einem chemischdefinierten Arzneistoff verbunden sind.

Fab-Fragmente (Fragment antigen-binding = Fab) bestehen aus den variablen Regionen beider Ketten, die durch die angrenzenden konstanten Regionen zusammengehalten werden. Sie können z.B. durch Behandlung mit einer Protease, wie beispielsweise Papain, aus konventionellen Antikörpern erzeugt werden oder aber auch durch DNA-Klonierung. Weitere Antikörperfragmente sind F(ab')₂-Fragmente, die durch proteolytischen Verdau mit Pepsin hergestellt werden können.

Durch Genklonierung können auch verkürzte Antikörperfragmente hergestellt werden, die nur aus den variablen Region der schweren (VH) und der leichten Kette (VL) bestehen. Diese werden als Fv-Fragmente (Fragment variable = Fragment des variablen Teils) bezeichnet. Da bei diesen Fv-Fragmenten die kovalente Verbindung über die Cysteinreste der konstanten Ketten nicht möglich ist, werden diese Fv-Fragmente oft anderweitig stabilisiert. Dazu werden die variablen Region der schweren und leichten Kette häufig mittels eines kurzen Peptidfragments von ca. 10 - 30 Aminosäuren, besonders bevorzugt 15 Aminosäuren, miteinander verknüpft. Auf diese Weise entsteht eine einzelne Polypeptidkette, in der VH und VL durch einen Peptidlinker miteinander verbunden sind. Solche Antikörperfragmente werden auch als *single-chain* Fv-Fragment (scFv) bezeichnet. Beispiele von scFv-Antikörpern sind bekannt und beschrieben, siehe z.B. Huston et al. (1988).

In den vergangenen Jahren wurden verschiedene Strategien entwickelt um multimere scFv-Derivate herzustellen. Die Intention besteht in der Erzeugung von rekombinanten Antikörpern mit verbesserten pharmakokinetischen Eigenschaften und verstärkter Bindungsavidität. Zur Erreichung der Multimerisierung der scFv-Fragmente werden diese als Fusionsproteine mit Multimerisierungsdomänen hergestellt. Als Multimerisierungsdomänen können dabei z.B. die CH3-Region eines IgGs oder Helixstrukturen ("*coiled coil structure*") wie die Leucin-Zipper-Domänen fungieren. In anderen Strategien wird die Interaktion zwischen den VH- und VL-Regionen des scFv-Fragments für eine Multimerisierung genutzt (z.B. Dia-, Tri- und Pentabodies).

Als "Diabody" bezeichnet ein Fachmann ein bivalentes homodimeres scFv-Derivat. Die Verkürzung des Peptidlinkers im scFv-Moleküle auf 5 - 10 Aminosäuren resultiert in der Bildung von Homodimeren durch Überlagerung von VHNL-Ketten. Die Diabodies können zusätzlich durch eingeführte Disulfidbrücken stabilisiert werden. Beispiele von Diabodies finden sich in der Literatur, z.B. bei Perisic et al. (1994).

Als "Minibody" bezeichnet der Fachmann ein bivalentes, homodimeres scFv-Derivat. Es besteht aus einem Fusionsprotein, das die CH3-Region eines Immunglobulins, vorzugsweise IgG, besonders bevorzugt IgG1, als Dimerisierungsregion enthält. Diese verbindet die scFv-Fragmente über eine Hinge-Region, ebenfalls von IgG, und eine Linker-Region. Beispiele solcher Minibodies sind bei Hu et al. (1996) beschrieben.

Mit "Triabody" bezeichnet der Fachmann ein trivalentes homotrimeres scFv-Derivat (Kortt et al., 1997). Die direkte Fusion von VH-VL ohne Verwendung einer Linkersequenz führt zur Ausbildung von Trimeren.

Bei den vom Fachmann als Mini-Antikörper bezeichneten Fragmenten, die eine bi-, tri- oder tetravalente Struktur haben, handelt es sich ebenfalls um Derivate von scFv-Fragmenten. Die Multimerisierung wird dabei über di-, tri- oder tetramere "coiled coil"-Strukturen erzielt (Pack et al., 1993 und 1995; Lovejoy et al., 1993).

Mit dem Begriff "Hilfsstoffe" sind Stoffe gemeint, die einer Formulierung, in der vorliegenden Erfindung einem Pulver, insbesondere einem sprühgetrockneten Pulver, zugefügt werden. Hilfsstoffe haben üblicherweise selbst keine pharmazeutische Wirkung und dienen dazu, die Formulierung des eigentlichen Wirksstoffs zu verbessern oder bezüglich eines bestimmten Aspekts (z.B. Lagerstabilität) zu optimieren.

Ein pharmazeutischer "Hilfsstoff" meint einen Teil eines Arzneimittels oder einer pharmazeutischen Zusammensetzung, und sorgt unter anderem dafür, dass der Wirkstoff an den Wirkort gelangt und dort freigesetzt wird. Hilfsstoffe haben drei Grundaufgaben: Trägerfunktion, Steuerung der Wirkstoff-Freigabe und Stabilitätserhöhung. Hilfsstoffe dienen auch der Herstellung von Arzneiformen, die sich dadurch in Wirkungsdauer oder - geschwindigkeit verändern.

Der Ausdruck "Aminosäure" meint Verbindungen, welche mindestens eine Amino- und mindestens eine Carboxylgruppe enthalten. Obwohl die Aminogruppe üblicherweise in α-Position zur Carboxylgruppe steht ist auch jede andere Anordnung im Molekül denkbar. Die Aminoäure kann auch weitere funktionelle Gruppen, wie z.B. Amino-, Carboxamid-, Carboxyl-, Imidazol-, Thiogruppen und andere Gruppen, enthalten. Verwendung finden Aminosäuren natürlichen oder synthetischen Ursprungs, razemisch oder optisch aktiv (D-, oder L-) inklusive verschiedener stereoisomerer Verhältnisse. Beispielsweise umfasst der Begriff Isoleucin sowohl D- Isoleucin, L- Isoleucin, razemisches Isoleucin und verschiedene Verhältnisse der beiden Enantiomere.

Mit dem Begriff "Peptid", "Polypeptid" oder "Protein" sind Polymere von Aminosäuren bestehend aus mehr als zwei Aminosäureresten gemeint.

Weiterhin sind mit dem Begriff "Peptid", "Polypeptid" oder "Protein" Polymere von Aminosäuren bestehend aus mehr als 10 Aminosäureresten gemeint.

Der Begriff Peptid, Polypeptid oder Protein wird als pseudonym verwendet und umfasst sowohl Homo- als auch Heteropeptide, also Polymere von Aminosäuren bestehend aus identischen oder verschiedenen Aminosäureresten. Ein "Di-Peptid" ist somit aus zwei peptidisch verknüpften Aminosäuren, ein "Tri-Peptid" aus drei peptidisch verknüpften Aminosäuren aufgebaut.

Der hier verwendete Begriff "Protein" meint Polymere von Aminosäuren mit mehr als 20 und insbesondere von mehr als 100 Aminosäureresten.

Der Begriff "kleines Protein" bezeichnet Proteine unter 50kD bzw. unter 30kD bzw. zwischen 5-50kD. Der Begriff "kleines Protein" bezeichnet weiterhin Polymere von Aminosäureresten mit weniger als 500 Aminosäureresten bzw. von weniger als 300 Aminosäureresten bzw. von Polymeren mit 50-500 Aminosäureresten. Bevorzugte kleine Proteine sind z.B. Wachstumsfaktoren wie "human growth hormone/ factor", Insulin, Calcitonin oder Ähnliches.

Der Begriff "Proteinstabilität" bedeutet Monomerengehalt über 90%, bevorzugt über 95%.

Der Ausdruck "Oligosaccharid" oder "Polysaccharid" meint Mehrfachzucker die zumindest aus drei monomeren Zuckermolekülen aufgebaut sind.

Der Ausdruck "% (w/w)" meint den prozentualen, auf die Masse bezogenen Anteil eines Wirkstoffes bzw. eines Hilfsstoffes im sprühgetrockneten Pulver. Hierbei wird der angegebene Anteil auf die Trockensubstanz des Pulvers bezogen. Die Restfeuchte im Pulver wird demnach nicht mit berücksichtigt.

Der Begriff "amorph" meint, dass die pulverförmigen Formulierung weniger als 10% kristalline Anteile enthalten, vorzugsweise weniger als 7%, weiter bevorzugt weniger als 5%, insbesondere weniger als 4, 3, 2, oder 1 %.

Der Begriff "inhalierbar" meint, dass die Pulver zur pulmonalen Applikation geeignet sind. Inhalierbare Pulver lassen sich mit Hilfe eines Inhalationsgerätes dispergieren und inhalieren, so dass die Partikel die Lunge erreichen und gegebenenfalls über die Alveolen systemische Wirkung entfalten können. Inhalierbare Partikel weisen beispielsweise eine mittlere Teilchengröße zwischen 0,4-30 µm (MMD = Mass median diameter), meistens zwischen 0,5-20 µm, bevorzugt zwischen 1-10 µm und/oder einen mittleren aerodynamischen Teilchendurchmesser (MMAD = Mass median aerodynamic diameter) zwischen 0,5-10 µm, vorzugsweise zwischen 0,5-7,5 µm, weiter bevorzugt zwischen 0,5-5,5 µm, noch weiter bevorzugt 1-5 µm und in besonders bevorzugter Weise zwischen 1-4,5µm bzw. 3-10µm auf.

"Mass Median Diameter" oder "MMD" ist eine Messgröße für die durchschnittliche Partikelgrößenverteilung. Die Ergebnisse werden ausgedrückt als Durchmesser der Volumensummenverteilung bei 50% Durchgangssumme. Die MMD-Werte lassen sich beispielhaft mittels Laserdiffraktometrie bestimmen, wobei natürlich auch jede andere übliche Methode verwendet werden kann (z.B. Elektronenmikroskopie, Zentrifugalsedimentation).

Der Begriff "mittlerer aerodynamischer Teilchendurchmesser" (= mass median aerodynamic diameter (MMAD)) gibt die aerodynamische Teilchengröße an, bei der normalerweise 50% der Teilchen des Pulvers einen kleineren aerodynamischen Durchmesser aufweisen. Als Referenzmethode zur Bestimmung des MMAD dient im Zweifel die in dieser Patentschrift angegebene Methode (vgl. hierzu: Kapitel BEISPIELE, Methode).

MMD und MMAD können voneinander abweichen, z.B. kann eine durch Sprühtrocknung entstandene Hohlkugel einen im Vergleich zum MMAD grösseren MMD aufweisen.

Der Begriff "Feinpartikelfraktion" (FPF) beschreibt den inhalierbaren Teil eines Pulvers bestehend aus Teilchen mit einer Teilchengröße von ≤ 5 µm MMAD. In gut dispergierbaren Pulvern beträgt die FPF mehr als 20%, vorzugsweise mehr als 30%, besonders bevorzugt mehr als 40%, noch weiter bevorzugt mehr als 50%, noch weiter bevorzugt mehr als 55%. Der in diesem Zusammenhang verwendete Begriff "Cut Off Diamenter" gibt an, welche Partikel bei der Bestimmung der FPF berücksichtigt werden. Eine FPF von 30% bei einem Cut Off Diameter von 5 µm (FPF₅) meint, dass zumindest 30% aller Partikel im Pulver einen mittleren aerodynamischen Teilchendurchmesser von kleiner 5 µm aufweisen.

Der Begriff " time of flight" ist die Bezeichnung für eine Standardmessmethode wie im Kapitel BEISPIELE näher beschrieben. Bei einer time of flight Messung wird der MMAD über die Bestimmung der Flugzeit eines Partikels für eine definierte Messstrecke ermittelt. Der MMAD korreliert mit der Flugzeit. Das heißt, dass Partikel mit einem großen MMAD eine größere Flugzeit benötigen als entsprechend kleinere Partikel. (vgl. hierzu: Kapitel BEISPIELE, Methode).

"Dispergierbar" meint flugtauglich. Grundvoraussetzung für eine Flugtauglichkeit eines Pulvers ist das Deagglomerieren des Pulvers in Einzelpartikel und das Verteilen der Einzelpartikel in Luft. Partikelagglomerate sind zu groß, um in die Lunge zu gelangen und sind demnach nicht geeignet für eine Inhalationstherapie.

Der Begriff "ausgebrachte Masse" gibt die Pulvermenge an, die bei Anwendung eines Inhalators ausgebracht wird. Die Ausbringung wird in diesem Fall zum Beispiel anhand einer Kapsel bestimmt, indem die Kapsel vor und nach Ausbringung gewogen wird. Die ausgebrachte Masse entspricht der Massendifferenz der Kapsel vor und nach Ausbringung.

Der Begriff "Trägerstoff" meint verglichen zum sprühgetrocknetem Pulver große Partikel. Durch diese Eigenschaft wird es ermöglicht, dass die sprühgetrockneten Pulver sich auf den Trägerstoff aufziehen. Werden beispielsweise sprühgetrocknete Partikel mit einem mittleren Durchmesser von ca. 5µm herstellt, so sollte der Trägerstoff eine mittlere Partikelgröße von 50-200µm haben. Typische Trägerstoffe sind Zucker und Polyole. Die Auswahl der Trägerstoffe ist jedoch nicht auf diese Stoffklassen beschränkt.

Der Begriff "mikroskalige Partikel" oder "mikroskalige Hilfsstoffe" bezeichnet Partikel, die im gleichen Größenbereich liegen wie die Partikel im sprühgetrockneten Pulver. Bei den mikroskaligen Partikeln handelt es sich bevorzugt um Partikel mit einer mittleren aerodynamischen Teilchengröße (MMAD) zwischen 0.5 - 10µm, 1 - 10µm, besonders bevorzugt 2 - 7.5µm. Mikroskalige Hilfsstoffe eignen sich besonders gut, um im Sprühtrockner Pulvermischungen herzustellen, da sich die Pulverkomponenten hierbei aerodynamisch ähnlich verhalten und somit Entmischungsprozesse zurückgedrängt werden. Daher eignen sich mikroskalige Hilfsstoffe bevorzugt zur Herstellung von Verdünnungen und Dosierungen, insbesondere bei hohen Anteilen von sprühgetrocknetem Pulver, insbesondere bei hohen Anteilen von sprühgetrocknetem, proteinhaltigem Pulver.

Der Begriff "Nanopartikel" oder "nanoskalige Partikel" meint verglichen zum sprühgetrocknetem Pulver sehr kleine Partikel. Durch diese Eigenschaft wird es ermöglicht, dass die sprühgetrockneten Pulver von nanoskaligen Partikeln beschichtet werden. Werden beispielsweise sprühgetrocknete Partikel mit einem mittleren Durchmesser von ca. 5µm herstellt, so sollte das Nanopartikel eine mittlere Partikelgröße von 1nm-500nm, beziehungsweise von 5nm-250nm, beziehungsweise 10nm-100nm haben.

"Aerosil®" bezeichnet Nanopartikel aus Siliziumdioxid (SiO2) bzw. aus modifiziertem, hydrophobisierten (Acylkette) Siliziumdioxid wie Aerosil® R812 von Degussa. Weitere Beispiele für Nanopartikel sind z.B. Titandioxid (TiO2).

Unter dem Begriff "bioabbaubare Nanopartikel" werden im Folgenden Nanopartikel verstanden, die im menschlichen oder tierischen Körper abgebaut werden können, bevorzugt ohne dass schädliche oder unnatürliche Abbauprodukte entstehen.

Der Begriff "Dosierung" bzw. "Dosierungen" meint die Menge eines Stoffes, insbesondere eines therapeutischen Wirkstoffes, die bei Anwendung eines Applikationsgerät, zum Beispiel ein Inhalator, ausgebracht wird. Maßgeblich für die Dosis ist der Stoffanteil, insbesondere Wirkstoffanteil, im Pulver sowie die ausgebrachte Menge an Pulver. Die ausgebrachte Dosis bei sprühgetrockneten Pulvern kann demnach entweder durch eine Veränderung des Wirkstoffanteils im sprühgetrockneten Pulver oder aber durch ein Zumischen bzw. Vermischen eines inerten, d.h. wirkstofffreien, Pulvers eingestellt werden.

Der Begriff "Verdünnung" meint eine verringerte Dosierung eines sprühgetrockneten Pulvers, insbesondere eines wirkstoffhaltigen sprühgetrockneten Pulvers.

Der Begriff "Mischungskomponente" meint die Substanz, die neben dem sprühgetrockneten Pulver in den Sprühtrockner als Pulver eingespeist wird. Bei der Mischungskomponente kann es sich um nanoskalige beziehungsweise mikroskalige Partikel bzw. Trägerstoffe handeln.

### ERFINDUNGSGEMÄSSE ZUSAMMENSETZUNGEN

Die vorliegende Erfindung betrifft ein Verfahren zur Mischung von sprühgetrocknetem Pulver mit Nanopartikeln, mikroskaligen Partikeln und/ oder mit Trägerstoffen dadurch gekennzeichnet, dass der Mischvorgang im Sprühtrockner erfolgt.

Das vorliegende Verfahren ist insbesondere dadurch gekennzeichnet, dass ein Transfer des Pulvers nach Sprühtrocknung in eine Mischapparatur entfällt.

Die vorliegende Erfindung betrifft zudem ein Verfahren zur Mischung von Pulvern gekennzeichnet durch folgende Schritte:
a. Versprühung / Zerstäubung einer Sprühlösung enthaltend eine oder mehrere zu versprühende Substanzen sowie optional einen oder mehrere Hilfsstoffe in ein Kompartiment,
b. Trocknung der entstandenen Tropfen in demselben Kompartiment wie in Schritt (a),
c. Einbringung eines oder mehrerer weiterer Pulver enthaltend Trägerstoffe oder Nanopartikel in dasselbe Kompartiment wie in Schritt (b) unter Bedingungen bei denen eine Mischung entsteht und
d. Sammlung der entstandenen Partikel.

Eine Mischung entsteht insbesondere dann, wenn im Rahmen der Schritte c oder d sowohl die sprühgetrockneten Partikel als auch die weiteren Pulverkomponenten deagglomeriert zusammengeführt werden. Dies geschieht bevorzugt bei einer pneumatischen Zerstäubung der in Schritt c hinzugefügten Trägerstoffe oder Nanopartikel. Ein bevorzugter Mischungsdruck bei dieser pneumatischen Zerstäubung ist 1,75 bar bei 1 mm oder 2 mm Spaltbreite der Dispergiereinheit.

Alternativ zur pneumatischen Zerstäubung über einen Mischspalt kann die Mischung insbesondere auch durch weitere Gasstrahldüsen, wie beispielweise die Venturidüse, bewirkt werden.

Alternativ zur pneumatischen Deagglomerierung der hinzugefügten Trägerstoffe und Nanopartikel kann auch eine Ultraschall- bzw. eine elektrostatisch-induzierte Deagglomerierung vorgenommen werden.

In einer speziellen Ausführungsform ist das erfindungsgemäße Verfahren dadurch gekennzeichnet, dass es sich
a. bei den sprühgetrockneten Pulvern um Pulver mit einer mittleren aerodynamischen Teilchengröße (MMAD) zwischen 0.5-10µm, 1-10µm, bevorzugt 2 - 7.5µm handelt,
b. bei den Nanopartikeln um Partikel mit einer mittleren Teilchengröße (MMD) von kleiner 500nm, kleiner 200nm bzw. um eine MMD zwischen 1nm-500nm, 5-250nm, bevorzugt 10-100nm,
c. bei den mikroskaligen Partikeln um Partikel mit einer mittleren aerodynamischen Teilchengröße (MMAD) zwischen 0.5-10µm, 1-10µm, bevorzugt 2 - 7.5µm handelt und
d. bei den Trägerstoffen um Stoffe mit einer mittleren Teilchengröße (MMD) von größer 50µm bzw zwischen 50-200µm, bevorzugt um 60-100µm handelt.

In einer bevorzugten Ausführungsform ist das erfindungsgemäße Verfahren dadurch gekennzeichnet, dass die Trägerstoffe einen Anteil von mindestens 30%(w/w) an Partikeln mit einer Partikelgröße kleiner 100µm haben.

In einer besonderen Ausführungsform ist das erfindungsgemäße Verfahren dadurch gekennzeichnet, dass mindestens 30% (w/w) der Trägerstoffe kleiner 100µm sind.

In einer weiteren Ausführungsform ist das Verfahren dadurch gekennzeichnet, dass es sich bei den Nanopartikeln oder bei den Trägerstoffen um Zucker, Polyole oder Aminosäuren handelt.

In einer bevorzugten Ausführungsform ist das Verfahren dadurch gekennzeichnet, dass es sich bei dem Trägerstoff um einen pharmazeutisch akzeptablen, kristallinen Hilfsstoff handelt, wie zum Beispiel einen kristallinen Zucker, beispielsweise Laktose Monohydrat, Glukose oder Chitosan oder um ein kristallines Polyol (beispielsweise Mannitol).

In einer weiteren Ausführungsform ist das Verfahren dadurch gekennzeichnet, dass es sich bei den Nanopartikeln um anorganische Verbindungen wie modifiziertes oder nicht modifiziertes Siliziumdioxid (SiO2), Titandioxid (TiO2) oder um Calciumcarbonat (CaCO3) handelt.

In einer besonders bevorzugten Ausführungsform ist das Verfahren dadurch gekennzeichnet, dass es sich bei den Nanopartikeln um bioabbaubare Nanopartikel handelt. Hierzu gehören beispielsweise nanoskalige Monosaccharide bzw. nanoskalige Polyole, wie Glukose oder Mannitol, nanoskalige Di-, Oligo- oder Polysaccharide, wie beispielsweise Laktose Monohydrat, Saccharose, Stärke, Amylose, Amylopektin, Stärkehydrolysate, Hydroxyethylstärke, Carrageenan, Chitosan, oder Dextrane, nanoskalige Aminosäuren wie beispielsweise Valin oder Glycin, nanoskalige Polymere wie beispielsweise Gelatine, Polyacrylate, Polymethacrylate, Polycyanoacrylate (z.B. Poly(isohexylcyanoacrylat), Poly(methylcyanoacrylat), Poly(ethylcyanoacrylat)), PLGA (Poly(lactic-co-glycolic acid), Polylactide, Polyglycolide, Polycaprolactone oder humanes Serumalbumin (HSA) oder nanoskalige Lipide, wie beispielsweise Tripalmitin-haltige oder Phosphatidylcholin-haltige Nanopartikel.

In einer weiteren speziellen Ausführungsform ist das Verfahren dadurch gekennzeichnet, dass es sich bei den Nanopartikeln nicht um Aerosil® (Siliziumdioxid) und / oder Titandioxid handelt.

In einer weiteren Ausführungsform ist das erfindungsgemäße Verfahren dadurch gekennzeichnet, dass das Kompartiment aus Schritt (a), (b) und (c) ein Sprühtrockner ist. In einer weiteren Ausführungsform ist das Verfahren dadurch gekennzeichnet, dass die Trocknung in Schritt (b) in einem Trocknungsturm erfolgt.

In einer speziellen Ausführungsform ist das Verfahren dadurch gekennzeichnet, dass Schritt (b) im Gleichstromverfahren durchgeführt wird.

In einer speziellen Ausführungsform ist das Verfahren dadurch gekennzeichnet, dass Schritt (b) nicht im Gegenstromverfahren durchgeführt wird.

In einer weiteren Ausführungsform ist das Verfahren dadurch gekennzeichnet, dass die Partikel in Schritt (d) in einem Zyklon gesammelt werden.

In einer weiteren Ausführungsform ist das erfindungsgemäße Verfahren dadurch gekennzeichnet, dass die Sprühlösung aus Schritt (a) entweder eine wässrige ist oder eine, die aus einem beliebigen pharmazeutisch akzeptablen organischen Lösungsmittel besteht.

In einer weiteren Ausführungsform ist das erfindungsgemäße Verfahren dadurch gekennzeichnet, dass das Trocknungsmedium in Schritt (b) entweder Luft oder Stickstoff ist.

In einer weiteren Ausführungsform ist das erfindungsgemäße Verfahren dadurch gekennzeichnet, dass bei der Trocknung in Schritt (b) die Eingangstemperatur des Trocknungsgases zwischen 50°C und 200°C liegt und die Auslasstemperatur des Trocknungsgases nach dem Trocknungsprozeß zwischen 25°C und 150°C liegt.

In einer weiteren Ausführungsform ist das erfindungsgemäße Verfahren dadurch gekennzeichnet, dass die Temperaturbelastung des sprühgetrockneten Pulvers durch Einblasen von kühler Luft nach der Trocknung (z.B. am Auslaß des Trocknungsturms) reduziert wird.

In einer weiteren Ausführungsform ist das erfindungsgemäße Verfahren dadurch gekennzeichnet, dass ein oder mehrere Trägerstoffe oder Nanopartikel über separate Dispergier- und Zudosierungseinheiten direkt in ein Kompartiment eingebracht werden.

In einer weiteren Ausführungsform ist das erfindungsgemäße Verfahren dadurch gekennzeichnet, dass ein oder mehrere Trägerstoffe oder Nanopartikel vorgemischt werden und dann gemeinsam über eine Dispergier- und Zudosierungseinheit direkt in den Sprühtrockner eingebracht werden.

In einer weiteren Ausführungsform ist das erfindungsgemäße Verfahren dadurch gekennzeichnet, dass kein zusätzlicher Energieeintrag zum Dispergieren des sprühgetrockenten Pulvers notwendig ist.

In einer weiteren Ausführungsform ist das erfindungsgemäße Verfahren dadurch gekennzeichnet, dass zwei Proteine zusammen gemischt werden.

In einer speziellen Ausführungsform sind die erfindungsgemäßen Verfahren dadurch gekennzeichnet, dass Verfahren zur Herstellung von Detergenzien oder von anorganischen Mischungen ausgeschlossen sind, beziehungsweise sind Verfahren ausgeschlossen, bei welchen durch Trocknung anorganische Pulver hergestellt werden, die anschließend mit einem weiteren Pulver vermischt werden.

In einer speziellen Ausführungsform sind die erfindungsgemäßen Verfahren dadurch gekennzeichnet, dass keine Detergenzien oder anorganischen Mischungen hergestellt werden bzw. keine Mischungen, bei welchen durch Trocknung anorganische Pulver hergestellt werden, die anschließend mit einem weiteren Pulver vermischt werden.

Die vorliegende Erfindung betrifft weiterhin ein Verfahren zur Beschichtung von sprühgetrockneten Partikeln mit Nanopartikeln dadurch gekennzeichnet, dass eines der beschriebenen erfindungsgemäßen Verfahren verwendet wird.

Die vorliegende Erfindung betrifft zudem ein Verfahren zur Herstellung von Zusammensetzungen oder Dosierungen, enthaltend einen definierten Anteil in Gewichtsprozent (w/w) an sprühgetrocknetem Pulver in der Pulvermischung, mittels Zumischung eines Trägerstoffes dadurch gekennzeichnet, dass eines der erfindungsgemäßen Verfahren verwendet wird.

In einer bevorzugten Ausführungsform sind die erfindungsgemäßen Verfahren dadurch gekennzeichnet, dass es sich bei dem sprühgetrockneten Pulver um ein proteinhaltiges Pulver handelt.

In einer bevorzugten Ausführungsform handelt es sich bei dem Protein um einen Antikörper.

In einer speziellen Ausführungsform handelt es sich bei der Zusammensetzung um eine Zusammensetzung zur Verwendung als Arzneimittel.

In einer bevorzugten Ausführungsform wird die Zusammensetzung als inhalatives Arzneimittel verwendet.

In einer weiteren Ausführungsform wird die erfindungsgemäße Zusammensetzung zur Herstellung eines Medikamentes zur Behandlung einer Lungenerkrankung oder einer systemischen Erkrankung verwendet.

Die Erfindung betrifft weiterhin Pulvermischungen dadurch gekennzeichnet, dass sie einem sprühgetrockneten Proteinanteil über 1 % (w/w), insbesondere über 30% (w/w), 35% (w/w), 40% (w/w), 45% (w/w), 50% (w/w), 55% (w/w), 60% (w/w), 65% (w/w), über 70% (w/w), über 80% (w/w) und über 90% (w/w) enthält und mindestens einen Nanopartikel oder einen Trägerstoff, wobei die Pulvermischung eine Feinpartikelfraktion über 15% (w/w), über 25% (w/w), über 35% (w/w), über 45% (w/w), über 55% (w/w) über 65% (w/w) aufweist.

In einer speziellen Ausführungsform ist die erfindungsgemäße Pulvermischung dadurch gekennzeichnet, dass es sich bei dem Proteinanteil um Antikörper handelt.

### BEISPIELE

### BEISPIEL 1

In diesem Beispiel wurde eine Sprühlösung hergestellt, die bezogen auf den Feststoffanteil 70% IgG2 und 30% Trehalose Dihydrat enthielt. Der Feststoffanteil der Lösung lag bei 3%. Die Sprühlösung wurde mit einem Büchi B-191 unter Verwendung eines sogenannten High Performance Cyclon (HPC) getrocknet. Verglichen zum Standardzyklon hat der HPC aufgrund des geringeren Durchmessers eine niedrigere Abscheidegrenze und dadurch eine bessere Abscheideeffzienz.

Die Trocknungsbedingungen waren:
Eingangstemperatur: 160°C
Sprührate der Lösung: 3,0 mL/min
Zerstäubergasrate: 700L/h

Die Herstellung der Mischungen erfolgte direkt im Trocknungsturm durch Einblasen von Laktose Monohydrat (Granulac 140) (siehe Abbildung 1A). Das Dispergieren der Laktose erfolgte durch Scherungswirkung an einem Spalt (Spaltbreite 1 mm). Der Dispergierdruck lag bei 1,75bar.

Es wurden 3 verschiedene Pulver beziehungsweise Pulvermischungen hergestellt.

**Tabelle 1**

| | Pulver 1 | Pulver 2 | Pulver 3 |
|---|---|---|---|
| Anteil sprühgetrocknetes Pulver in der Mischung (%w/w) | 100 | 70 | 10 |
| Anteil Granulac 140 in der Mischung (%w/w) | 0 | 30 | 90 |
| Ausgebrachte Masse, % | 69,5 | 79,6 | 98,5 |
| Feinpartikelfraktion, % | 12,3 | 24,2 | 28,7 |

Sowohl bei Pulver 2 also auch bei Pulver 3 konnte durch Zumischen von Granulac 140 die aerodynamischen Eigenschaften (FPF und ausgebrachte Masse) der Mischungen verglichen zum sprühgetrocknetem Pulver ohne Granulac 140 (Pulver 1) verbessert werden (Siehe Abbildung 2). Die Feinpartikelfraktion wurde nasschemisch bezogen auf den Wirkstoff in der Kapsel vor Ausbringung bestimmt. Die ausgebrachte Masse ergibt sich aus der Massendifferenz vor und nach Ausbringung des Pulver aus dem Pulverinhalator (HandiHaler®).

Abbildung 3 zeigt eine rasterelektronenmikroskopische Aufnahme der Pulvermischung Pulver 2.

### BEISPIEL 2

In diesem Beispiel wurde die Homogenität der ausgebrachten Dosis an einer Mischung aus sprühgetrocknetem Pulver und einem Trägerstoff (Granulac 140) ermittelt. Die Parameter für die Sprühtrocknung wurden analog wie im Beispiel 1 beschrieben eingestellt.

Zusammensetzung der Pulver:

**Tabelle 2**

| | ST60 | ST63 |
|---|---|---|
| Sprühgetrocknetes Pulver | 70% (w/v) IgG2 / 30% (w/v) Trehalose | 70% (w/v) IgG2 / 30% (w/v) Trehalose |
| Trägerstoff | Granulac 140 | - |
| Massenverhaltnis | 9/1 | - |
| Trägerstoff / sprühgetrocknetes Pulver | | |

**Tabelle 3**

| Pulver ST60 | Dosis in Prozent bezogen auf die Wirkstoffeinwaage in der Kapsel | Dosis in Prozent bezogen auf die ausgebrachte Proteinmenge | Differenz [%absolut] | Ausgebrachte Masse in Prozent |
|---|---|---|---|---|
| Messung 1 | 87,0 | 86,3 | -0,7 | 97,7 |
| Messung 2 | 89,9 | 89,7 | -0,2 | 97,1 |
| Messung 3 | 92,8 | 91,8 | -1,0 | 98,0 |
| Messung 4 | 112,4 | 112,1 | -0,2 | 97,1 |
| Messung 5 | 119,2 | 120,0 | 0,8 | 96,3 |
| Messung 6 | 104,0 | 104,9 | 1,0 | 96,0 |
| Messung 7 | 86,3 | 86,5 | 0,1 | 96,8 |
| Messung 8 | 93,2 | 93,3 | 0,1 | 96,8 |
| Messung 9 | 111,5 | 112,9 | 1,5 | 95,7 |
| Messung 10 | 103,8 | 102,5 | -1,3 | 98,2 |
| Min | 86,3 | 86,3 | -1,3 | 95,7 |
| Max | 119,2 | 120,0 | 1,5 | 98,2 |
| rel. Standardabweichung | 11,7 | 12,2 | | 0,9 |

**Tabelle 4**

| Pulver ST63 | Dosis in Prozent bezogen auf die Wirkstoffeinwaage in der Kapsel | Dosis in Prozent bezogen auf die ausgebrachte Proteinmenge | Differenz [%absolut] | Ausgebrachte Masse in Prozent |
|---|---|---|---|---|
| Messung 1 | 90,0 | 109,1 | 19,1 | 74,4 |
| Messung 2 | 94,2 | 107,4 | 13,3 | 79,1 |
| Messung 3 | 106,5 | 107,3 | 0,8 | 89,5 |
| Messung 4 | 104,2 | 95,3 | -9,0 | 98,7 |
| Messung 5 | 96,2 | 101,4 | 5,2 | 85,6 |
| Messung 6 | 116,6 | 115,2 | -1,4 | 91,3 |
| Messung 7 | 78,3 | 77,9 | -0,4 | 90,6 |
| Messung 8 | 129,7 | 97,8 | -31,9 | 119,6 |
| Messung 9 | 112,3 | 113,1 | 0,8 | 89,5 |
| Messung 10 | 72,0 | 75,5 | 3,4 | 86,1 |
| Min | 72,0 | 75,5 | -31,9 | 74,4 |
| Max | 129,7 | 115,2 | 19,1 | 119,6 |
| rel. Standardabweichung | 17,5 | 13,8 | | 13,6 |

Durch die Herstellung der Mischung bestehend aus 90% Granulac 140 und 10% sprühgetrocknetem Pulver konnte verglichen zum sprühgetrockneten Pulver die Homogenität beziehungsweise die Gleichförmigkeit der Dosis verbessert werden. Wie aus Tabelle 3 und 4 zu ersehen ist, sind sowohl die ausgebrachten Dosierungen bezogen auf die Wirkstoffeinwaage in der Kapsel als auch auf die ausgebrachte Menge an Protein homogener. Des weiteren verbessert sich durch die Herstellung der Mischung die Ausbringung des Pulver aus der Kapsel gravierend.

### BEISPIEL 3

In diesem Beispiel wurde die Reproduzierbarkeit der Herstellung von Pulvermischungen im Sprühtrockner überprüft. Hierzu wurde eine Pulverrezeptur analog wie in Bespiel 1 beschrieben dreifach hergestellt. Die Zuführung des Granulac 140 erfolgte bei einem Dispergierdruck von 1,75bar und einer Spaltbreite von 2mm. Die Tabelle 5 zeigt die auf die Einwaage bezogenen Feinpartikelfraktionen sowie die ausgebrachten Massen an Pulver aus dem Inhalator. Beide Messparameter zeigen eine sehr enge Schwankungsbreite. Das bedeutet, dass der Mischvorgang im Sprühtrockner sehr präzise durchgeführt werden kann.

**Tabelle 5**

| Herstellnummer | ST60 | ST61 | ST62 | |
|---|---|---|---|---|
| Sprühgetrocknetes Pulver | 70% (w/v) IgG2 / 30% (w/v) Trehalose | | | |
| Trägerstoff | Granulac 140 | | | |
| Massenverhaltnis | 9/1 | | | |
| Trägerstoff / sprühgetrocknetes Pulver | - | | | |
| FPF [%] | 28,7 | 24,4 | 24,6 | |
| Ausgebrachte Masse [%] | 98,5 | 97,0 | 98,4 | |

### BEISPIEL 4

Dieses Beispiel zeigt verschiedene Mischungen bestehend aus sprühgetrocknetem Pulver und Aerosil R812. In Abbildung 4 ist ein Beispiel für ein mit Aerosil beschichtetes sprühgetrocknetes Pulver zu sehen.

In Abbildung 5 werden die erzielten Feinpartikelfraktionen der verschiedenen Mischungen in Abhängigkeit des Dispergierdrucks dargestellt.

Durch das Beschichten mit Aerosil R812 kann die Feinpartikelfraktion erhöht werden.

### BEISPIEL 5

Dieses Beispiel zeigt eine Mischung von sprühgetrocknetem Pulver mit dem hydrophobisiertem Siliziumdioxid (Aerosil R 972, Degussa) sowie eine Mischung aus hochdispersem hydrophilem Siliziumdioxid (Merck, Darmstadt, CAS-Nr.: 7631-86-9). Die Prozessbedingungen der Sprühtrocknung sind in Tabelle 6 dargestellt. Die Zuführung der hydrophoben bzw. der hydrophilen nanoskaligen Mischungskomponente erfolgte pneumatisch. Hierzu wurde ein Vorratsgefäß (Gesamtvolumen 1,1 L) mit 200mL nanoskaligem Pulver befüllt. Oberhalb des Pulverbettes wurden 0,5L/min Luft tangential in das Vorratsgefäß eingebracht. Zur Homogenisierung des Pulverbettes wurde zusätzlich das Pulver mechanisch gerührt (300 Umdrehungen / Minute). Das Pulver wurde anschließend über eine Venturidüse in den Sprühtrockner eingespeist. Der Vordruck der Venturidüse lag bei 2 bar. Zwischen dem Vorratsgefäß und der Venturidüse war ein weiteres Gefäß zwischengeschaltet. In diesem Gefäß wurden gröbere Partikelagglomerate abgeschieden. Optional besteht die Möglichkeit über dieses Gefäß weitere Mischluft aber auch weitere Partikelaerosole in den Sprühtrockner einzubringen.

**Tabelle 6**

| | |
|---|---|
| Sprühtrockner | Büchi B 191 |
| Eingangstemperatur | 150°C |
| Trocknungsgasrate | 100% |
| Sprührate | 5 mL/min |
| Zerstäubergasrate | 700L / h |
| Zyklon | Standard |

Zur Herstellung der Sprühlösung wurden 0,9gTrehalose Dihydrat in ca. 70mL Wasser gelöst. Nach dem Lösen wurden 15,4mL IgG1-haltige Lösung (Proteinkonzentration: 104mg/mL) zugegeben und auf 100mL mit Wasser aufgefüllt. Der mittlere Proteinanteil im Pulver nach Sprühtrocknung ohne Zuführung einer Mischungskomponente lag bei 58%. Nach Mischung mit hydrophoben SiO₂ verringerte sich der Proteinanteil im Pulver auf 47%. Bei hydrophilem SiO₂ lag der Proteinanteil bei 54%.

In Tabelle 7 sind die aerodynamischen Eigenschaften der verschiedenen hergestellten Pulver dargestellt. Die Messungen erfolgten analog wie in Abbildung 2 beschrieben. Abweichend zur dort beschriebenen Durchführung wurde die Feinpartikelfraktion gravimetrisch durch Wägung der eingesetzten Kapsel vor und nach Ausbringung des Pulvers in die Messeinrichtung bestimmt.

**Tabelle 7**

| Mischungskomponente | Ohne Zugabe einer Mischungskomponente / Herstellcharge: N45 | Hydrophobes SiO₂ / Herstellcharge: N34 | Hydrophiles SiO₂ / Herstellchargen: N40 und N43 |
|---|---|---|---|
| FPF, % | 26 | 59 | 50 |
| EM, % | 80 | 94 | 92 |
| MMAD, µm | 2,7 | 3,4 | 4,3 |

Die Erhöhung der gravimetrisch ermittelten FPF durch Zugabe von nanoskaligen Partikeln von 33% FPF bei hydrophobem SIO₂ bzw. von 29% FPF bei hydrophilem SiO₂ ist wesentlich auf eine Optimierung der Oberflächenbeschaffenheit der sprühgetrockneten Pulver zurückzuführen, da der Mischanteil der zusätzlichen Komponente kleiner 11% und somit die Erhöhung der FPF nicht hauptsächlich in einen erhöhten Massenanteil an inhalierbaren SiO₂ im Pulver begründet ist.

### BEISPIEL 6

Dieses Beispiel zeigt die Möglichkeit, über ein direktes Vermischen von mikroskaligen Hilfsstoffen mit dem sprühgetrockneten Pulver den Proteinanteil im Pulver und damit die die Wirkstoffdosis einzustellen.

Die Sprühbedingungen zur Herstellung des sprühgetrockneten Pulvers sind in Tabelle 8 beschrieben.

Zur Herstellung der Sprühlösung wurden 0,9g Trehalose Dihydrat in ca. 70mL Wasser gelöst. Nach dem Lösen wurden 15,4mL IgG1-haltige Lösung (Proteinkonzentration: 104mg/mL) zugegeben und auf 100mL mit Wasser aufgefüllt. Der Proteinanteil im Pulver nach Sprühtrocknung ohne Zuführung einer Mischungskomponente lag bei 60%.

**Tabelle 8**

| | |
|---|---|
| Sprühtrockner | Büchi B-191 |
| Eingangstemperatur | 150°C |
| Trocknungsgasrate | 100% |
| Sprührate | 5 mL/min |
| Zerstäubergasrate | 700L / h |
| Zyklon | Standard |

Als Mischungskomponente wurde Laktose-Monohydrat eingesetzt, welches durch Mahlung mikronisiert wurde. Nach dem Mikronisieren hatte der Zucker einen MMAD von 3,9 µm und eine gravimetrisch ermittelte Feinpartikelfraktion von 14%.

Die Dosierung des mikroniserten Laktose Monohydrates erfolgte über einen Schneckendosierer (ZD9F, Firma Three-Tec). Das ausgeworfene Pulver wurde mit einem Luftstrom von 20Umin einer Venturidüse zugeführt. Der Vordruck der Venturidüse lag bei 0,69bar. Wie in Tabelle 9 beschrieben, wurden 2 Pulvermischungen mit unterschiedlichen Dosierraten des Schneckendosieres hergestellt. Die Feinpartikelfraktion beziehungsweise die Proteinmenge in der Feinpartikelfraktion wurde nasschemisch bestimmt. Hierzu wurden 3 Kapseln in den Impactor Inlet (Typ 3306 / Firma TSI) ausgebracht und anschließend der Filter nach dem Impaktordüse analysiert. Die Methode ist in Abbildung 2 beschrieben. Als Rekonstititionsmediums wurde ein Puffer, bestehend aus 25 mM Histidin / 1,6 mM Glycin, pH 6,0, eingesetzt.

Das Mischungsverhältnis zwischen dem sprühgetrockneten Pulver und dem mikronisierten Laktose Monohydrat ist in Tabelle 9 dargestellt. Beim Pulver 2 ergibt sich beispielsweise ein Proteinanteil im Pulver von 38,8% durch ein Mischen der beiden Komponenten im Verhältnis 62% (w/w) sprühgetrocknetes Pulver und 38 % (w/w) mikronisierte Laktose Monohydrat.

Wie in Tabelle 9 dargestellt, konnte der Proteinanteil in der Feinpartikelfraktion durch ein Vermischen mit der Mischungskomponente signifikant verringert werden.

Die Proteinanteile im Pulver und damit das Mischungsverhältnis ist nach Ausbringung mit dem Handihaler in der Feinpartikelfraktion annähernd identisch zu der Ausgangsmischung. In Tabelle 10 sind die Proteinanteile der Ausgangsmischung und in der ermittelten Feinpartikelfraktion dargestellt. Der Proteinanteil in der Feinpartikelfraktion ergibt sich aus dem nasschemisch ermittelten Proteinanteil in der FPF bezogen auf der Pulvermenge in der FPF. Hieraus ergibt sich, dass im Besonderen mikroskalige Hilfsstoffe wie beispielsweise mikronisiertes Laktose Monohydrat sich gut eignen, um im Sprühtrockner Pulvermischungen herzustellen, da die Pulverkomponenten sich aerodynamisch ähnlich verhalten und somit Entmischungsprozesse zurückgedrängt werden.

**Tabelle 9**

| | Pulver 1 | Pulver 2 | Pulver 3 |
|---|---|---|---|
| Dosierrate ZD9F, U/min | Keine Zumischung | 5 | 8 |
| Proteinanteil im Pulver nach Herstellung, % | 60,2 | 38,8 | 28,5 |
| FPF, % | 22 | 17 | 14 |
| Proteinmenge in der Feinpartikelfraktion, mg | 5,5 | 3,9 | 2,2 |
| Mischungsverhältnis sprühgetrocknetes Pulver/ Mischungskomponente % w/w | - | 62/38 | 47/53 |

**Tabelle 10**

| | Proteinanteil der Pulvermischung nach Sprühtrocknung, % | Proteinanteil der Pulvermischung in der Feinpartikelfraktion, % |
|---|---|---|
| Pulver 2 | 37,8 | 37,9 |
| Pulver 3 | 28,5 | 29,5 |

### BEISPIEL 7

Es wurde eine Sprühlösung entsprechend der Einwaagen wie in Tabelle 11 beschrieben hergestellt. Die Sprühbedingungen zur Herstellung des sprühgetrockneten Pulvers sind in Tabelle 12 beschrieben.

**Tabelle 11**

| | Einwaage in Gramm für 1 Liter gereinigtes Wasser |
|---|---|
| Trehalose Dihydrat | 84 |
| L-Histidine | 0,68 |
| L-Histidin HCL Monohydrat | 3,27 |
| Polysorbat 80 | 0,2 |
| EDTA | 0,1 |
| Bovines Serum Albumin | 0,82 |

**Tabelle 12**

| | |
|---|---|
| Sprühtrockner | Büchi B-191 |
| Eingangstemperatur | 150°C |
| Trocknungsgasrate | 100% |
| Sprührate | 5 mUmin |
| Zerstäubergasrate | 700L / h |
| Zyklon | Standard |

Das sprühgetrocknete Pulver wurde im Sprühtrockner mit verschiedenen Pharmatosen (DMV) (Siehe Tabelle 13) vermischt. Die Zudosierung erfolgte mit einem Schneckendosierer (ZD9F, Three-Tec) mit 10U/min. Das ausgeworfene Pulver wurde direkt mit einem Luftstrom von 20 L/min in den Sprühtrockner eingebracht.

Wie in Tabelle 13 ersichtlich, ist das erzielte Mischungsergebnis maßgeblich von der Partikelgröße der eingesetzten Mischungskomponente abhängig. Speziell gröbere Trägerstoffe wie Pharmatose 50M und Pharmatose 90M eignen sich weniger für das In-Line Mischen, da es hier verstärkt zu Entmischungsprozessen kommt. Die eingesetzte Mischungskomponente sollte höchstens die Partikelgröße entsprechend Pharmatose 125 M haben. Kleinere Partikel sind im Besonderen bei größeren Zugaben der Mischungskomponente vorzuziehen.

**Tabelle 13**

| | Pulver 1 | Pulver 2 | Pulver 3 | Pulver 4 |
|---|---|---|---|---|
| Mischungskomponente | Keine Zumischung | Pharmatose 50M | Pharmatose 90M | Pharmatose 125M |
| Anteil der Mischungskomponente im Pulver, % (w/w) | - | 65 | 63 | 62 |
| Theoretische Proteinbeladung | - | 0,37 | 0,40 | 0,41 |
| Gemessene Proteinbeladung im Pulver, % (w/w) | 1,07 | 0,08 | 0,12 | 0,26 |
| Wiederfindung, % | - | 21 | 30 | 64 |

## Patentansprüche

1. Verfahren zur Mischung von sprühgetrocknetem Pulver mit Nanopartikeln, mikroskaligen Partikeln und/ oder mit Trägerstoffen **dadurch gekennzeichnet, dass** der Mischvorgang im Sprühtrockner erfolgt.

2. Verfahren germäß Anspruch 1 **dadurch gekennzeichnet, dass** es folgende Schritte umfasst:
a. Versprühung /Zerstäubung einer Sprühlösung enthaltend eine oder mehrere zu versprühende Substanzen sowie optional einen oder mehrere Hilfsstoffe in ein Kompartiment,
b. Trocknung der entstandenen Tropfen in demselben Kompartiment wie in Schritt (a),
c. Einbringung eines oder mehrerer weiterer Pulver enthaltend Nanopartikel, mikroskalige Partikel und/ oder Trägerstoffe in dasselbe Kompartiment wie in Schritt (b) unter Bedingungen bei denen eine Mischung entsteht und
d. Sammlung der entstandenen Partikel.

3. Verfahren gemäß Anspruch 1 bis 2 **dadurch gekennzeichnet, dass** es sich
a. bei den sprühgetrockneten Pulvern um Pulver mit einer mittleren aerodynamischen Teilchengröße (MMAD) zwischen 0.5-10µm, 1-10µm, bevorzugt 2 - 7.5µm handelt,
b. bei den Nanopartikeln um Partikel mit einer mittleren Teilchengröße (MMD) von kleiner 500nm, kleiner 200nm bzw. um eine MMD zwischen 1nm-500nm, 5-250nm, bevorzugt 10-100nm,
c. bei den mikroskaligen Partikeln um Partikel mit einer mittleren aerodynamischen Teilchengröße (MMAD) zwischen 0.5-10µm, 1-10µm, bevorzugt 2 - 7.5µm handelt und
d. bei den Trägerstoffen um Stoffe mit einer mittleren Teilchengröße (MMD) von größer 50µm bzw zwischen 50-200µm, bevorzugt um 60-100µm handelt.

4. Verfahren gemäß Anspruch 3 **dadurch gekennzeichnet, dass** die Trägerstoffe einen Anteil von mindestens 30%(w/w) an Partikeln mit einer Partikelgröße kleiner 100µm haben.

5. Verfahren gemäß einem der Ansprüche 1 bis 4 **dadurch gekennzeichnet, dass** es sich bei den Nanopartikeln um bioabbaubare Nanopartikel handelt ausgewählt aus der Gruppe bestehend aus:
Nanoskalige Monosaccharide, nanoskalige Polyole wie Glukose und Mannitol, nanoskalige Di-, Oligo- und Polysaccharide, wie beispielsweise Laktose Monohydrat, Saccharose, Stärke, Amylose, Amylopektin, Stärkehydrolysate, Hydroxyethylstärke, Carrageenan, Chitosan und Dextrane, nanoskalige Aminosäuren wie Valin und Glycin, nanoskalige Polymere wie beispielsweise Gelatine, Polyacrylate, Polymethacrylate, Polycyanoacrylate (z.B. Poly(isohexylcyanoacrylat), Poly(methylcyanoacrylat), Poly(ethylcyanoacrylat)), PLGA (Poly(lactic-co-glycolic acid)), Polylactide, Polyglycolide, Polycaprolactone und humanes Serumalbuminn (HSA) und nanoskalige Lipide, wie Tripalmitin-haltige und Phosphatidylcholin-haltige Nanopartikel.

6. Verfahren gemäß Anspruch 2 bis 5 **dadurch gekennzeichnet, dass** das Kompartiment aus Schritt (a), (b) und (c) ein Sprühtrockner ist.

7. Verfahren gemäß Anspruch 2 bis 6 **dadurch gekennzeichnet, dass** Schritt (b) im Gleichstromverfahren durchgeführt wird.

8. Verfahren gemäß einem der Ansprüche 2 bis 7 **dadurch gekennzeichnet, dass** das Trocknungsmedium in Schritt (b) entweder Luft oder Stickstoff ist und dass bei der Trocknung in Schritt (b) die Eingangstemperatur des Trocknungsgases zwischen 50°C und 200°C liegt und die Auslasstemperatur des Trocknungsgases nach dem Trocknungsprozeß zwischen 25°C und 150°C liegt.

9. Verfahren gemäß einem der Ansprüche 1 bis 8 **dadurch gekennzeichnet, dass** die Temperaturbelastung des sprühgetrockneten Pulvers durch Einblasen von kühler Luft nach der Trocknung (z.B. am Auslaß des Trocknungsturms) reduziert wird.

10. Verfahren gemäß einem der Ansprüche 1 bis 9 **dadurch gekennzeichnet, dass** ein oder mehrere Trägerstoffe oder Nanopartikel vorgemischt werden und dann gemeinsam über eine Dispergier- und Zudosierungseinheit direkt in den Sprühtrockner eingebracht werden.

11. Verfahren gemäß einem der Ansprüche 1 bis 10 zur Beschichtung von sprühgetrockneten Partikeln mit Nanopartikeln.

12. Verfahren gemäß einem der Ansprüche 1 bis 10 zur Herstellung von Zusammensetzungen oder Dosierungen.

13. Verfahren nach einem der Ansprüche 1 bis 12 **dadurch gekennzeichnet, dass** es sich bei dem sprühgetrockneten Pulver um ein proteinhaltiges Pulver handelt.

14. Verfahren nach Anspruch 12 oder 13 **dadurch gekennzeichnet, dass** die Zusammensetzung ein Arzneimittel ist.

15. Verfahren nach Anspruch 12 oder 13 **dadurch gekennzeichnet, dass** die Zusammensetzung ein Arzneimittel zur Behandlung einer Lungenerkrankung oder einer systemischen Erkrankung ist.

16. Verfahren nach einem der Ansprüche 12 bis 15 **dadurch gekennzeichnet, dass** die Zusammensetzung einen sprühgetrockneten Proteinanteil über 1% (w/w), insbesondere über 30% (w/w), 35% (w/w), 40% (w/w), 45% (w/w), 50% (w/w), 55% (w/w), 60% (w/w), 65% (w/w), über 70% (w/w), über 80% (w/w) und über 90% (w/w) enthält und mindestens einen Nanopartikel, einen mikroskaligen Partikel oder einen Trägerstoff, wobei die Pulvermischung eine Feinpartikelfraktion über 15% (w/w), über 25% (w/w), über 35% (w/w), über 45% (w/w), über 55% (w/w), über 65% (w/w) aufweist.

## Claims

1. Method of mixing spray-dried powder with nanoparticles, microscale particles and/or with carriers, **characterised in that** the mixing process takes place in the spray dryer.

2. Method according to claim 1, **characterised in that** it comprises the following steps:
a. spraying/atomising a spray solution containing one or more substances that are to be sprayed, as well as optionally one or more excipients, into a compartment,
b. drying the resulting drops in the same compartment as in step (a),
c. introducing one or more other powders containing nanoparticles, microscale particles and/or carriers into the same compartment as in step (b) under conditions in which a mixture is formed and
d. collecting the particles formed.

3. Method according to claims 1 to 2, **characterised in that**
a. the spray-dried powders are powders with a mean aerodynamic particle size (MMAD) of between 0.5-10 µm, 1-10 µm, preferably 2-7.5 µm,
b. the nanoparticles are particles with a mean particle size (MMD) of less than 500 nm, less than 200 nm or an MMD of between 1 nm -500 nm, 5-250 nm, preferably 10-100 nm,
c. the microscale particles are particles with a mean aerodynamic particle size (MMAD) of between 0.5-10 µm, 1-10 µm, preferably 2-7.5 µm, and
d. the carriers are substances with a mean particle size (MMD) of more than 50 µm or between 50-200 µm, preferably 60-100 µm.

4. Method according to claim 3, **characterised in that** the carriers have a proportion of at least 30% (w/w) of particles with a particle size of less than 100 µm.

5. Method according to one of claims 1 to 4, **characterised in that** the nanoparticles are biodegradable nanoparticles selected from among:
nanoscale monosaccharides, nanoscale polyols such as glucose and mannitol, nanoscale di-, oligo- and polysaccharides, such as for example lactose monohydrate, saccharose, starch, amylose, amylopectin, hydrolysed starch, hydroxyethylstarch, carrageenan, chitosan and dextrans, nanoscale amino acids such as valine and glycine, nanoscale polymers such as for example gelatine, polyacrylates, polymethacrylates, polycyanoacrylates (e.g. poly(isohexylcyanoacrylate), poly(methylcyanoacrylate), poly(ethylcyanoacrylate)), PLGA (poly(lactic-co-glycolic acid), polylactides, polyglycolides, polycaprolactones and human serum albumin (HSA) and nanoscale lipids, such as tripalmitin-containing and phosphatidylcholine-containing nanoparticles.

6. Method according to claim 2 to 5, **characterised in that** the compartment of step (a), (b) and (c) is a spray dryer.

7. Method according to claim 2 to 6, **characterised in that** step (b) is carried out by the cocurrent method.

8. Method according to one of claims 2 to 7, **characterised in that** the drying medium in step (b) is either air or nitrogen and during the drying in step (b), the entry temperature of the drying gas is between 50°C and 200°C and the exit temperature of the drying gas after the drying process is between 25°C and 150°C.

9. Method according to one of claims 1 to 8, **characterised in that** the temperature loading of the spray-dried powder is reduced by blowing in cool air after the drying (e.g. at the exit from the drying tower).

10. Method according to one of claims 1 to 9, **characterised in that** one or more carriers or nanoparticles are pre-mixed and then introduced together into the spray dryer directly through a dispersing and metering unit.

11. Method according to one of claims 1 to 10 for coating spray-dried particles with nanoparticles.

12. Method according to one of claims 1 to 10 for preparing compositions or dosages.

13. Method according to one of claims 1 to 12, **characterised in that** the spray-dried powder is a protein-containing powder.

14. Method according to claim 12 or 13, **characterised in that** the composition is a medicament.

15. Method according to claim 12 or 13, **characterised in that** the composition is a medicament for the treatment of a pulmonary disease or a systemic disease.

16. Method according to one of claims 12 to 15, **characterised in that** the composition has a spray-dried protein content of more than 1% (w/w), particularly more than 30% (w/w), 35% (w/w), 40% (w/w), 45% (w/w), 50% (w/w), 55% (w/w), 60% (w/w), 65% (w/w), more than 70% (w/w), more than 80% (w/w) and more than 90% (w/w) and comprises at least a nanoparticle, a microscale particle or a carrier, the powder mixture having a fine particle fraction of more than 15% (w/w), more than 25% (w/w), more than 35% (w/w), more than 45% (w/w), more than 55% (w/w), more than 65% (w/w).

## Revendications

1. Procédé de mélange d'une poudre séchée par pulvérisation comportant des nanoparticules, des particules à l'échelle du micron et/ou des substances de support, **caractérisé en ce que** le processus de mélange s'effectue dans le séchoir par pulvérisation.

2. Procédé selon la revendication 1, **caractérisé en ce qu'**il comprend les étapes suivantes :
a. pulvérisation/vaporisation d'une solution de pulvérisation contenant une ou plusieurs substances à pulvériser et éventuellement, un ou plusieurs adjuvants dans un compartiment,
b. séchage des gouttes obtenues dans le même compartiment que celui de l'étape (a),
c. insertion d'une ou plusieurs autres poudres contenant des nanoparticules, des particules à l'échelle du micron et/ou des substances de support dans le même compartiment que celui de l'étape (b) dans des conditions dans lesquelles un mélange est formé et
d. récolte des particules obtenues.

3. Procédé selon la revendication 1 à 2, **caractérisé en ce que**
a. les poudres séchées par pulvérisation sont des poudres présentant une taille aérodynamique moyenne de particule (MMAD) entre 0,5 et 10 µm, 1 et 10 µm, de préférence, 2 à 7,5 µm,
b. les nanoparticules sont des particules présentant une taille moyenne de particule (MMD) inférieure à 500 nm, inférieure à 200 nm ou une MMD entre 1 nm et 500 nm, 5 et 250 nm, de préférence, 10 et 100 nm,
c. les particules à l'échelle du micron sont des particules présentant une taille aérodynamique moyenne de particule (MMAD) entre 0,5 et 10 µm, 1 et 10 µm, de préférence, 2 et 7,5 µm et
d. les substances de support sont des substances présentant une taille moyenne de particule (MMD) supérieure à 50 µm ou entre 50 et 200 µm, de préférence de 60 à 100 µm.

4. Procédé selon la revendication 3, **caractérisé en ce que** les substances de support ont une proportion d'au moins 30 % (p/p) de particules présentant une taille de particule inférieure à 100 µm.

5. Procédé selon l'une des revendications 1 à 4, **caractérisé en ce que** les nanoparticules sont des nanoparticules biodégradables choisies dans le groupe consistant en :
monosaccharides à l'échelle du nanomètre, polyols à l'échelle du nanomètre tels que le glucose et le mannitol, di-, oligo- et polysaccharides à l'échelle du nanomètre tels que par exemple le lactose monohydraté, le saccharose, les amidons, l'amylose, l'amylopectine, les hydrolysats d'amidon, l'amidon d'hydroxyéthyle, le carraghénane, le chitosan et le dextrane, acides aminés à l'échelle du nanomètre tels que la valine et la glycine, polymères à l'échelle du nanomètre tels que par exemple, la gélatine, les polyacrylates, les polyméthacrylates, les polycyanoacrylates (par exemple, poly(isohexyl-cyanoacrylate), le poly(méthylcyano-acrylate), le poly(éthylcyanoacrylate)), le PLGA (poly(acide lactique co-glycolique), les polylactides, les polyglycolides, les polycaprolactones et en sérumalbumine humaine (HSA) et en lipides à l'échelle du nanomètre tels que les nanoparticules contenant de la tripalmitine et de la phosphatidylcholine.

6. Procédé selon les revendications 2 à 5, **caractérisé en ce que** le compartiment des étapes (a), (b) et (c) est un séchoir par pulvérisation.

7. Procédé selon les revendications 2 à 6, **caractérisé en ce que** l'étape (b) est réalisée dans un procédé à courant continu.

8. Procédé selon l'une des revendications 2 à 7, **caractérisé en ce que** le milieu de séchage à l'étape (b) est l'air ou l'azote et **en ce que**, lors du séchage de l'étape (b), la température d'entrée du gaz de séchage se situe entre 50° C et 200° C et la température de sortie du gaz de séchage après le processus de séchage se situe entre 25° C et 150° C.

9. Procédé selon l'une des revendications 1 à 8, **caractérisé en ce que** la charge thermique de la poudre séchée par pulvérisation est réduite par insufflation d'air plus froid après le séchage (par exemple, à la sortie de la tour de séchage).

10. Procédé selon l'une des revendications 1 à 9, **caractérisé en ce qu'**une ou plusieurs substances de support ou nanoparticules sont prémélangées et ensuite, sont intégrées conjointement, par une unité de dispersion et d'addition, directement dans le séchoir par pulvérisation.

11. Procédé selon l'une des revendications 1 à 10, pour le revêtement de particules séchées par pulvérisation avec des nanoparticules.

12. Procédé selon l'une des revendications 1 à 10, pour la production de compositions ou de dosages.

13. Procédé selon l'une des revendications 1 à 12, **caractérisé en ce que** la poudre séchée par pulvérisation est une poudre contenant des protéines.

14. Procédé selon la revendication 12 ou 13, **caractérisé en ce que** la composition est un médicament.

15. Procédé selon la revendication 12 ou 13, **caractérisé en ce que** la composition est un médicament pour le traitement d'une maladie pulmonaire ou d'une maladie systémique.

16. Procédé selon l'une des revendications 12 à 15, **caractérisé en ce que** la composition contient une proportion de protéine séchée par pulvérisation supérieure à 1 % (p/p), en particulier, supérieure à 30 % (p/p), de 35 % (p/p), 40 % (p/p), 45 % (p/p), 50 % (p/p), 55 % (p/p), 60 % (p/p), 65 % (p/p), supérieure à 70 % (p/p), supérieure à 80 % (p/p) et supérieure à 90 % (p/p) et présente au moins une nanoparticule, une particule à l'échelle du micron ou une substance de support, le mélange de poudre présentant une fraction de particules fines supérieure à 15 % (p/p), supérieure à 25 % (p/p), supérieure à 35 % (p/p), supérieure à 45 % (p/p), supérieure à 55 % (p/p), supérieure à 65 % (p/p).
